(19) Europäisches Patentamt
European Patent Office
Office européen des brevets

(11) **EP 1 888 229 B1**

(12) **EUROPÄISCHE PATENTSCHRIFT**

(45) Veröffentlichungstag und Bekanntmachung des Hinweises auf die Patenterteilung:
**03.08.2011 Patentblatt 2011/31**

(21) Anmeldenummer: **05813596.3**

(22) Anmeldetag: **28.11.2005**

(51) Int Cl.:
*B01J 23/22* (2006.01)          *B01J 27/198* (2006.01)
*B01J 37/02* (2006.01)          *C07C 51/265* (2006.01)

(86) Internationale Anmeldenummer:
**PCT/EP2005/012703**

(87) Internationale Veröffentlichungsnummer:
**WO 2006/125468 (30.11.2006 Gazette 2006/48)**

(54) **MEHRLAGEN-KATALYSATOR ZUR HERSTELLUNG VON PHTHALSÄUREANHYDRID**

MULTI-LAYERED CATALYST FOR PRODUCING PHTHALIC ANHYDRIDE

CATALYSEUR MULTICOUCHE PERMETTANT DE PRODUIRE DE L'ANHYDRIDE PHTALIQUE

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **22.05.2005 PCT/EP2005/005548**

(43) Veröffentlichungstag der Anmeldung:
**20.02.2008 Patentblatt 2008/08**

(73) Patentinhaber: **SÜD-CHEMIE AG**
**80333 München (DE)**

(72) Erfinder:
• **GÜCKEL, Christian**
**85567 Grafing (DE)**
• **NIEDERMEIER, Markus**
**83043 Bad Aibling (DE)**
• **ESTENFELDER, Marvin**
**76199 Karlsruhe (DE)**

(74) Vertreter: **Stolmár, Matthias et al**
**Stolmár Scheele & Partner**
**Patentanwälte**
**Blumenstrasse 17**
**80331 München (DE)**

(56) Entgegenhaltungen:
**EP-A- 0 286 448          WO-A-00/12214**
**WO-A-99/61433          WO-A-2004/103561**
**WO-A-2005/115615          WO-A-2005/115616**
**WO-A1-2006/092304          WO-A1-2006/092304**
**WO-A1-2006/125467          WO-A1-2006/125467**

Bemerkungen:
Die Akte enthält technische Angaben, die nach dem Eingang der Anmeldung eingereicht wurden und die nicht in dieser Patentschrift enthalten sind.

EP 1 888 229 B1

## Beschreibung

Die Erfindung betrifft einen Mehrlagen-Katalysator, d.h. einen Katalysator mit drei oder mehr unterschiedlichen Lagen (Schichten) zur Herstellung von Phthalsäureanhydrid (PSA) durch Gasphasenoxidation von o-Xylol und/oder Naphtalin, wobei der Aktivmassegehalt von der ersten, zur Gaseintrittsseite hin gelegenen Katalysatorlage zu der zur Gasaustrittsseite hin gelegenen Katalysatorlage abnimmt.

Die großtechnische Produktion von Phthalsäureanhydrid wird durch die katalytische Gasphasenoxidation von o-Xylol und/oder Naphthalin erzielt. Zu diesem Zwecke wird ein für die Reaktion geeigneter Katalysator in einen Reaktor, vorzugsweise einen sogenannten Rohrbündelreaktor, indem eine Vielzahl von Rohren parallel angeordnet sind, gefüllt und von oben oder unten mit einem Gemisch aus dem (den) Kohlenwasserstoff(en) und einem sauerstoffhaltigen Gas, beispielsweise Luft, durchströmt. Aufgrund der starken Wärmebildung solcher Oxidationsreaktionen ist es nötig, die Reaktionsrohre zur Vermeidung von sogenannten Hot Spots ("Heißen Flecken") mit einem Wärmeträgermedium zu umspülen und somit die entstandene Wärmemenge abzuführen. Diese Energie kann zur Produktion von Dampf genutzt werden. Als Wärmeträgermedium dient in der Regel eine Salzschmelze und hier vorzugsweise ein eutektisches Gemisch aus $NaNO_2$ und $KNO_3$.

Ebenso kann man zur Unterdrückung der ungewollten Hot Spots einen strukturierten Katalysator in das Reaktionsrohr füllen, wodurch sich beispielsweise zwei oder drei Katalysatorlagen aus unterschiedlich zusammengesetzten Katalysatoren ergeben können. Solche Systeme sind als solche bereits aus der EP 1 082 317 B1 oder der EP 1 084 115 B1 bekannt.

Die schichtweise Anordnung der Katalysatoren hat auch den Zweck, den Gehalt an unerwünschten Nebenprodukten, d.h. Verbindungen, die in einem möglichen Reaktionsmechanismus von o-Xylol zu Phthalsäureanhydrid vor dem eigentlichen Wertprodukt stehen, im Roh-PSA so gering wie möglich zu halten. Zu diesen unerwünschten Nebenprodukten zählen hauptsächlich die Verbindungen o-Tolylaldehyd und Phthalid. Die Weiteroxidation dieser Verbindungen zu Phthalsäureanhydrid steigert zudem die Selektivität bzgl. des eigentlichen Wertprodukts.

Neben den oben angesprochenen Unteroxidationsprodukten treten bei der Reaktion auch Überoxidationsprodukte auf. Dazu gehören Maleinsäureanhydrid, Citraconsäureanhydrid, Benzoesäure und die Kohlenoxide. Eine gezielte Unterdrückung der Bildung dieser ungewünschten Nebenprodukte zu Gunsten des Wertprodukts führt zu einem weiteren Anstieg der Produktivität und Wirtschaftlichkeit des Katalysators.

Aus der EP 1 084 115 ist bekannt: ein Verfahren zur Herstellung von Phthalsäureanhydrid durch katalytische Gasphasenoxidation von Xylol und/oder Naphthalin mit einem molekularen Sauerstoff enthaltenden Gas in einem Festbett bei erhöhter Temperatur undmittels mindestens drei in Schichten übereinander angeordneter Schalenkatalysatoren, auf deren Kern aus Trägermaterialeine Schicht aus katalytisch aktiven Metalloxiden aufgebracht ist, dadurch gekennzeichnet, dass die Katalysatoraktivität von Schicht zu Schicht von der Gaseintrittsseite zur Gasaustrittsseite ansteigt, wobei die Aktivität der Katalysatoren der einzelnen Schichten so eingestellt wird, dass der am geringsten aktive Katalysator einen geringeren Aktivmassegehalt und gegebenenfalls zusätzlich mehr Alkalimetall, ausgewählt aus der Gruppe bestehend aus Kalium, Rubidium und Cäsium, als Dotierung als der Katalysator der nächsten Schicht aufweist und der darauf folgende noch aktivere Katalysator die gleiche Aktivmassenmenge und noch weniger Alkalimetall als Dotierung oder eine größere Aktivmassenmenge und gegebenenfalls weniger Alkalimetall als Dotierung als der Katalysator der zweiten Schicht aufweist, mit der Maßgabe, dass

a) der am geringsten aktive Katalysator auf nicht porösem Trägermaterial 5 bis 9 Gew.-%, bezogen auf den gesamten Katalysator, Aktivmasse aufweist, enthaltend 3 bis 8 Gew.-% $V_2O_5$, 0 bis 3,5 Gew.-% $Sb_2O_3$, 0 bis 0,3 Gew.-% P, 0,1 bis 0,5 Gew.-% Alkali (berechnet als Alkalimetall) und als Rest $TiO_2$ in Anatasform mit einer BET-Oberfläche von 18 bis 22 $m^2$/g,

b) der nächst aktivere Katalysator bei sonst gleicher Zusammensetzung wie Katalysator (a) einen um 1 bis 5 Gew.-% (absolut) höheren Aktivmassegehalt hat und der Alkaligehalt um 0 bis 0,25 Gew.-% (absolut) geringer ist und

c) der aktivste Katalysator bei sonst gleicher Zusammensetzung wie (a) einen um 1 bis 5 Gew.-% (absolut) höheren Aktivmassegehalt als für (a) hat und der Alkaligehalt um 0,15 bis 0,4 Gew.-% (absolut) geringer als für (a) ist.

Nachteilig an den dort angegebenen erfindungsgemäßen Katalysatoren ist, dass trotz des Einsatzes solcher strukturierten Katalysatoren noch immer sehr hohe Anteile an dem unerwünschten Nebenprodukt Phthalid im Roh-PSA enthalten ist. Dem Fachmann ist klar, dass eine destillative Trennung der beiden Produkte nur unter Verlusten des eigentlichen Wertproduktes möglich ist. Des weiteren sind die PSA-Ausbeuten noch zu verbessern.

Es besteht daher ein ständiger Bedarf nach verbesserten mehrlagigen (mehrschichtigen) Katalysatoren zur Herstellung von Phthalsäureanhydrid.

Aufgabe der vorliegenden Erfindung war es daher, einen verbesserten Katalysator zur Herstellung von Phthalsäureanhydrid durch Gasphasenoxidation von o-Xylol und/oder Naphthalin bereitzustellen, der die Nachteile des Stands der Technik vermeidet und insbesondere eine hohe Selektivität und Aktivität auch nach langer Betriebszeit ermöglicht.

Diese Aufgabe wird durch den Mehrlagen-Katalysator gemäß Anspruch 1 gelöst. Bevorzugte Ausführungsformen sind in den Unteransprüchen angegeben.

So wurde überraschend gefunden, dass sich besonders vorteil-hafte Mehrlagen-Katalysatoren herstellen lassen, wenn der Mehrlagen-Katalysator aus mindestens drei unterschiedlichen Lagen zusammengesetzt ist, wobei der Aktivmassegehalt von der ersten, zur Gaseintrittsseite hin gelegenen Katalysatorlage zu der zur Gasaustrittsseite hin gelegenen Katalysatorlage abnimmt. Dabei hat sich als wesentlich herausgestellt, dass die erste Katalysatorlage einen Aktivmassegehalt zwischen etwa 7 und 12 Gew.-%, insbesondere zwischen etwa 8 und 11 Gew.-%, aufweist, die zweite Katalysatorlage einen Aktivmassegehalt zwischen etwa 6 und 11 Gew.-%, insbesondere zwischen etwa 7 und 10 Gew.-%, aufweist, und die dritte bzw. letzte Katalysatorlage einen Aktivmassegehalt zwischen etwa 5 und 10 Gew.-%, insbesondere zwischen etwa 6 und 9 Gew.-%, aufweist.

Die Ausdrücke erste, zweite bzw. dritte Katalysatorlage werden im Zusammenhang mit der vorliegenden Erfindung wie folgt verwendet: als erste Katalysatorlage wird die zur Gaseintrittsseite hin gelegene Katalysatorlage bezeichnet. Zur Gasaustrittsseite hin sind im erfindungsgemäßen Mehrlagen-Katalysator noch zwei weitere Katalysatorlagen enthalten, die als zweite bzw. dritte Katalysatorlage bezeichnet werden. Die dritte Katalysatorlage liegt dabei näher zur Gasaustrittsseite als die zweite Katalysatorlage.

Nach einer besonders bevorzugten erfindungsgemäßen Ausführungsform weist der erfindungsgemäße Mehrlagen-Katalysator drei Katalysatorlagen auf. Dann liegt die dritte Katalysatorlage an der Gasaustrittsseite. Die Anwesenheit von zusätzlichen Katalysatorlagen gasstromab der ersten Katalysatorlage ist jedoch nicht ausgeschlossen. Beispielsweise kann nach einer erfindungsgemäßen Ausführungsform der dritten Katalysatorlage wie hierin definiert noch eine vierte Katalysatorlage (mit einem gleichen oder noch geringeren Aktivmassegehalt als die dritte Katalysatorlage) nachfolgen.

Erfindungsgemäß kann der Aktivmassegehalt zwischen der ersten und der zweiten Katalysatorlage und/oder zwischen der zweiten und der dritten Katalysatorlage abnehmen.

Nach einer besonders bevorzugten erfindungsgemäßen Ausführungsform nimmt der Aktivmassegehalt zwischen der zweiten und der dritten Katalysatorlage ab. Es versteht sich, dass von der Gaseintrittsseite zur Gasaustrittsseite in der Abfolge der Katalysatorlagen der Aktivmassegehalt nie zunimmt, sondern allenfalls gleich bleibt. Nach einer weiteren bevorzugten erfindungsgemäßen Ausführungsform nimmt der Aktivmassegehalt von Katalysatorlage zu Katalysatorlage in Gasstromrichtung, d.h. von der ersten zur letzten Katalysatorlage hin ab.

Es wird angenommen, ohne dass die Erfindung auf die Richtigkeit dieser Annahme beschränkt wäre, dass sich durch die mit den unterschiedlichen Aktivmassengehalten verknüpften unterschiedlichen Schichtdicken der katalytisch aktiven Masse in den einzelnen Lagen, besonders bevorzugt den abnehmenden Schichtdicken der katalytisch aktiven Masse von der ersten zur dritten Lage zum einen die Reaktion von o-Xylol bis hin zum PSA in der ersten und gegebenenfalls zweiten Lage beeinflusst wird, und des weiteren in der dritten Lage mit der noch dünneren Schicht an aktiver Masse die verbleibenden Unteroxidationsprodukte wie z.B. Phthalid zu PSA, jedoch nicht PSA zu den sogenannten Überoxidationsprodukten, wie z.B. $CO_x$ oxidiert werden. Dadurch wird über die gesamte strukturierte Packung die maximale Produktivität bei der Oxidation von o-Xylol zu PSA bei einem minimalen Anteil an den sogenannten unerwünschten Nebenprodukten erzielt.

Nach einer bevorzugten erfindungsgemäßen Ausführungsform nimmt dabei die BET-Oberfläche von der ersten, zur Gaseintrittsseite hin gelegenen Katalysatorlage zu der zur Gasaustrittsseite hin gelegenen Katalysatorlage (insbesondere der dritten oder vierten Katalysatorlage) zu. Dadurch können überraschend besonders gute Katalysatorleistungen erzielt werden. Bevorzugte Bereiche für die BET-Oberfläche sind 15 bis 25 $m^2$/g für die erste Katalysatorlage, 15 bis 25 $m^2$/g für die zweite Katalysatorlage und 25 bis 45 $m^2$/g für die dritte (bzw. letzte) Katalysatorlage.

Allgemein wird erfindungsgemäß bevorzugt, dass die BET-Oberfläche der ersten Katalysatorlage geringer ist als die BET-Oberfläche der dritten (bzw. letzten) Katalysatorlage. Besonders vorteilhafte Katalysatoren werden auch erhalten, wenn die BET-Oberfläche der ersten und der zweiten Katalysatorlage gleich sind, während die BET-Oberfläche der dritten Katalysatorlage demgegenüber größer ist. Insbesondere (jedoch nicht nur) soweit mehr als drei Katalysatorlagen vorhanden sind, ist es nach einer bevorzugten erfindungsgemäßen Ausführungsform auch vorteilhaft, dass die BET-Oberfläche der letzten, zur Gasaustrittsseite hin gelegenen Katalysatorlage größer ist, als die BET-Oberfläche der näher zur Gaseintrittsseite hin gelegenen Katalysatorlagen. Nach einer weiteren Ausführungsform kann dabei die BET-Oberfläche aller Katalysatorlagen, bis auf die letzte, zur Gasaustrittsseite hin gelegenen Katalysatorlage, gleich sein.

Die Katalysatoraktivität zur Gaseintrittsseite ist nach einer bevorzugten erfindungsgemäßen Ausführungsform geringer als die Katalysatoraktivität zur Gasaustrittsseite hin. Danach nimmt die Katalysatoraktivität von der ersten zur letzten Lage bei dieser Ausführungsform zu.

Das verwendete $TiO_2$ (Anatas-Modifikation) (in allen Katalysatorlagen) weist einen Gehalt an Na von weniger als 0,3 Gew.-%, insbesondere weniger als 0,2 Gew.-%, bevorzugt weniger als 0,15 Gew.-%, weiter bevorzugt weniger als 0,02 Gew.-%, weiter bevorzugt weniger als 0,015 Gew.-% auf. Vorzugsweise gelten die vorstehenden Grenzwerte für Na und K. Nach einem weiteren bevorzugten Aspekt der Erfindung ist der Anteil an Alkaliverunreinigungen (Gesamtalkaligehalt) des eingesetzten $TiO_2$, bestimmt als die Summe der Lithium-, Natrium-, Kalium-, Rubidium- und Cäsiumverunreinigungen, kleiner als 1000 ppm, insbesondere kleiner 500 ppm, besonders bevorzugt kleiner 300 ppm. Ein Verfahren zur Bestimmung des Anteils an Alkaliverunreinigungen des eingesetzten $TiO_2$ ist nachstehend vor den Beispielen angegeben (DIN ISO 9964-3). Der vorstehende Gesamtalkaligehalt des $TiO_2$ ermöglicht eine exakte Einstellung des Alkali-Promotorgehaltes des Katalysators.

Der Anteil an Alkaliverunreinigungen kann, wie dem Fachmann geläufig, im Bedarfsfall durch Waschen z.B. mit verdünnter Salpetersäure bei erhöhter Temperatur gesenkt werden, um den bevorzugten Bereich von kleiner 1000 ppm zu erreichen. Z.B. kann das $TiO_2$ in 0,1 M $HNO_3$ aufgeschlämmt und unter Rückfluß und unter Agitation bei 90°C über Nacht gewaschen, anschliessend filtriert und dreifach mit bidestilliertem Wasser gewaschen und bei 150°C an Luft getrocknet werden. Anschließend wird der Anteil an Alkaliverunreinigungen erneut bestimmt, und, soweit zu hoch, die vorstehende Prozedur wiederholt.

Es wurde weiterhin überraschend gefunden, dass sich die erfindungsgemäßen Mehrlagen- bzw. Mehrschicht-Katalysatoren mit abnehmendem Aktivmassegehalt zur Herstellung von Phthalsäureanhydrid besonders vorteilhaft einsetzen lassen, wenn die einzelnen Katalysatorlagen in einem bestimmten Längenverhältnis zueinander vorliegen.

Allgemein lässt sich feststellen, dass besonders gute Phthalsäureanhydridausbeuten erreicht werden können, wenn das Verhältnis der Längen der ersten und zweiten Schicht zwischen 1,2 und 5, und das der Längen der ersten und dritten Schicht ebenfalls zwischen 1,2 und 5 liegt. Bevorzugt liegt das Verhältnis der Längen der ersten und zweiten Schicht zwischen 2 und 4, besonders bevorzugt zwischen 2,5 und 3,5. Bevorzugt liegt das Verhältnis der Längen der ersten und dritten Schicht zwischen 2 und 4, besonders bevorzugt zwischen 2,5 und 3,5.

So weist nach einer besonders bevorzugten erfindungsgemäßen Ausführungsform die erste, zur Gaseintrittsseite hin gelegene Katalysatorlage einen Längenanteil, bezogen auf die Gesamtlänge des Katalysatorbettes, von mindestens 40%, insbesondere mindestens 45%, besonders bevorzugt mindestens 50% auf. Insbesondere bevorzugt wird, dass der Anteil der ersten Katalysatorlage an der Gesamtlänge des Katalysatorbettes zwischen 40 und 70%, insbesondere zwischen 40 und 55%, besonders bevorzugt zwischen 40 und 52% liegt.

Die zweite Lage nimmt vorzugsweise etwa 10 bis 40%, insbesondere etwa 10 bis 30% der Gesamtlänge des Katalysatorbettes ein. Weiterhin wurde überraschend gefunden, dass ein Verhältnis der Länge der dritten Katalysatorlage zur Länge der zweiten Katalysatorlage zwischen etwa 1 und 2, insbesondere zwischen 1,2 bis 1,7 besonders bevorzugt zwischen 1,3 und 1,6, besonders gute Ergebnisse im Hinblick auf die Wirtschaftlichkeit wie die Effizienz der Rohstoffnutzung und Produktivität des Katalysators liefert.

Es hat sich.gezeigt, dass durch die vorstehende Wahl der Längenanteile der einzelnen Katalysatorlagen, insbesondere im Zusammenspiel mit den abnehmenden Aktivrnassegehalten wie vorstehend definiert, eine besonders günstige Positionierung des Hot Spots, insbesondere in der ersten Lage, und eine gute Temperaturführung zur Vermeidung zu hoher Hot Spot-Temperaturen auch bei längerer Betriebsdauer des Mehrlagen-Katalysators ermöglicht wird. Dadurch wird die Ausbeute, insbesondere bezogen auf die Lebensdauer des Mehrlagen-Katalystors, verbessert. Es wird angenommen, ohne dass die Erfindung auf diese Annahme beschränkt wäre, dass durch das vorstehende Lagenlängenverhältnis der einzelnen Katalysatorlagen zueinander noch innerhalb der zweiten Katalysatorlage eine praktisch vollständige Umsetzung des eingesetzten o-Xylols erfolgt und somit in der dritten Katalysatorlage mit den oben beschriebenen Vorteilen das sogenannte "Produktpolishing", d.h. das Reinigen des Reaktionsgases von unerwünschten Nebenprodukten durch Oxidation zum eigentlichen Wertprodukt erfolgt. Des weiteren ist dem Fachmann bekannt, dass nach einer bestimmten Laufzeit solche Katalysatoren im Bereich des Hot Spots (in der Regel in der ersten Lage) deaktivieren. Durch diese Deaktivierung kommt es zu einer Verlagerung der Reaktion in die zweite aktivere Lage, was zu sehr hohen Hot Spot-Temperaturen und den damit verbundenen Problemen in Bezug auf Selektivität und Anlagensicherheit führt. Durch die im erfindungsgemäßen Mehrlagen-Katalysator gewählten Lagenverhältnisse, ist eine maximale Verweilzeit des Hot Spots in der ersten Lage mit den bekannten Vorteilen gewährleistet, wobei gleichzeitig durch die erfindungsgemäße Länge der zweiten und dritten Lage ein minimaler Anteil an unerwünschten Nebenprodukten bei gleichzeitig maximaler Ausbeute an eigentlichem Wertprodukt gewährleistet wird.

Es wurde auch gefunden, dass die hierin definierten Lagenlängenverhältnisse auch bei anderen Mehrlagenkatalysatoren, d.h. die nicht die erfindungsgemäße Abnahme des Aktivmasseanteils aufweisen, Vorteile zeigt. Dies gilt neben den Katalysatoren zur Herstellung von Phthalsäureanhydrid (PSA) durch Gasphasenoxidation von o-Xylol und/ oder Naphtalin auch allgemein für andere Mehrlagenkatalysatoren zur Gasphasenoxidation von Kohlenwasserstoffen.

Das Temperaturmanagement bei der Gasphasenoxidation von o-Xylol zu Phthalsäureanhydrid ist dem Fachmann aus dem Stand der Technik hinreichend bekannt, wobei beispielsweise auf die DE 100 40 827 A1 verwiesen werden kann.

Nach einer weiteren bevorzugten Ausführungsform enthält die Aktivmasse (katalytisch aktive Masse) des erfindungsgemäßen Mehrlagen-Katalysators Titandioxid mit einer spezifischen BET-Oberfläche und vorzugsweise einer spezifischen Porenradienverteilung. Dabei wurde überraschend gefunden, dass sich bei der Verwendung eines Titandioxids, worin mindestens 25%, insbesondere mindestens etwa 40%, besonders bevorzugte mindestens etwa 50%, am meisten bevorzugt mindestens etwa 60% des gesamten Porenvolumens durch Poren mit einem Radius zwischen 60 und 400 nm gebildet werden, besonders vorteilhafte Mehrlagen-Katalysatoren erzeugen lassen.

Nach einer weiteren bevorzugten Ausführungsform wird $TiO_2$ verwendet, das eine Primärkristallitgröße (Primärpartikelgröße) von mehr als etwa 210 Angström, vorzugsweise mehr als etwa 250 Angström, weiter bevorzugt mindestens 300 Angström, insbesondere mindestens etwa 350 Angström, weiter bevorzugt mindestens 390 Angström aufweist. So wurde gefunden, dass solche $TiO_2$-Primärkristallite mit der vorstehenden (Mindest-)Größe die Herstellung von besonders vorteilhaften Mehrlagen-Katalysatoren ermöglichen. Bevorzugt liegt die Primärkristallitgröße unter 900 Angström, insbesondere unter 600 Angström, weiter bevorzugt unter 500 Angström. Die vorstehende Primärkristallitgröße ermöglicht offenbar, ohne dass die Erfindung auf diese Annahme beschränkt wäre, die Ausbildung einer nicht zu kompakten, sondern offenporigen Struktur des Titandioxids im Katalysator. Ein Verfahren zur Bestimmung der Primärkristallitgröße ist im nachstehenden Methodenteil angegeben.

Nach einer weiteren bevorzugten Ausführungsform wird $TiO_2$ verwendet, das eine Schüttdichte von weniger als 1,0 g/ml, insbesondere weniger als 0,8 g/ml, besonders bevorzugt weniger als etwa 0,6 g/ml aufweist. Am meisten bevorzugt sind $TiO_2$-Materialien mit einer Schüttdichte von nicht mehr als etwa 0,55 g/ml. Ein Verfahren zur Bestimmung der Schüttdichte ist im nachstehenden Methodenteil angegeben. Es wurde somit gefunden, dass die Verwendung eines Titandioxids mit einer Schüttdichte wie vorstehend definiert, die Herstellung besonders leistungsfähiger Mehrlagen-Katalysatoren ermöglicht. Es wird angenommen, ohne dass die Erfindung hierauf beschränkt wäre, dass die Schüttdichte hier ein Maß für eine besonders günstige Struktur der im Katalysator zur Verfügung gestellten $TiO_2$-Oberfläche ist, wobei durch die lockere, nicht zu kompakte Struktur besonders günstige Reaktionsräume sowie Zuführ- und Ableitwege für die Reaktanden bzw. Reaktionsprodukte bereitgestellt werden.

Es wird angenommen, ohne dass die Erfindung auf die Richtigkeit dieser theoretischen Annahme beschränkt wäre, dass sich durch die Verwendung des Titandioxids mit den hierin beschriebenen Eigenschaften in einem Katalysator besonders vorteilhafte Reaktionsräume für die gewünschten Umsetzungen, insbesondere im Porengefüge, erzielen lassen. Gleichzeitig werden bei Verwendung der erfindungsgemäßen $TiO_2$-Matrix vorteilhafte Zuführwege für die Edukte zu den reaktiven Zentren auf der Oberfläche der $TiO_2$-Matrix sowie Ableitwege für die Reaktionsprodukte bereitgestellt.

Im Allgemeinen wird bei der Verwendung des erfindungsgemäßen Mehrlagen-Katalysators zur Herstellung von Phthalsäureanhydrid ein Gemisch aus einem molekularen Sauerstoff enthaltenden Gas, beispielsweise Luft, und dem zu oxidierenden Ausgangsmaterial (insbesondere o-Xylol und/oder Naphtalin) durch einen Festbettreaktor, insbesondere einen Rohrbündelreaktor, der aus einer Vielzahl parallel angeordneter Rohre bestehen kann, geleitet. In den Reaktorrohren befindet sich jeweils eine Schüttung aus mindestens einem Katalysator. Auf die Vorzüge einer Schüttung aus mehreren (unterschiedlichen) Katalysatorlagen wurde oben bereits eingegangen.

Beim Einsatz der erfindungsgemäßen Katalysatoren zur Herstellung von Phthalsäureanhydrid durch Gasphasenoxidation von o-Xylol und/oder Naphthalin wurde überraschend festgestellt, dass mit den erfindungsgemäßen Katalysatoren sehr gute PSA-Ausbeuten bei sehr geringen Anteilen an Phthalid erzielt werden.

Nach einer bevorzugten erfindungsgemäßen Ausführungsform weist das eingesetzte $TiO_2$ eine BET-Oberfläche von mindestens 15, vorzugsweise zwischen 15 und 60 $m^2/g$, insbesondere zwischen etwa 15 und 45 $m^2/g$ und besonders bevorzugt zwischen 15 und 30 $m^2/g$ auf. Die BET-Oberfläche des eingesetzten $TiO_2$ bestimmt weitgehend die BET-Oberfläche der Katalysatorlage. Nach einer Ausführungsform der Erfindung wird somit die BET-Oberfläche der Katalysatorlage mit der des jeweils eingesetzten $TiO_2$ gleichgesetzt.

Weiterhin wird bevorzugt, dass bis zu 80%, bevorzugt bis zu 75%, insbesondere bis zu 70% des gesamten Porenvolumens des $TiO_2$ durch Poren mit einem Radius zwischen 60 und 400 nm gebildet werden.

Die Bestimmung der hierin angegebenen Porenvolumina bzw. -anteile erfolgt, soweit nicht anders angegeben, mittels Quecksilberporosimetrie (gemäß DIN 66133). Die Angabe des Gesamtporenvolumens bezieht sich dabei in der vorliegenden Beschreibung jeweils auf das gesamte mittels Quecksilberporosimetrie gemessene Porenvolumen zwischen 7500 und 3,7 nm Porenradiengröße.

Poren mit einem Radius von mehr als 400 nm stellen bevorzugt weniger als etwa 30%, insbesondere weniger als etwa 22%, besonders bevorzugt weniger als 20% des gesamten Porenvolumens des eingesetzten $TiO_2$ dar.

Weiterhin bevorzugt wird, dass etwa 50 bis 75%, insbesondere etwa 50 bis 70%, besonders bevorzugt 50 bis 65% des gesamten Porenvolumens des $TiO_2$ durch Poren mit einem Radius von 60 bis 400 nm, und vorzugsweise etwa 15 bis 25% des gesamten Porenvolumens durch Poren mit einem Radius von mehr als 400 nm gebildet werden.

Bezüglich der kleineren Porenradien wird bevorzugt, dass weniger als 30%, insbesondere weniger als 20% des gesamten Porenvolumens des $TiO_2$ durch Poren mit einem Radius von 3,7 bis 60 nm gebildet werden. Ein hier besonders bevorzugter Bereich beträgt für diese Porengröße etwa 10 bis 30% des gesamten Porenvolumens, insbesondere 12 bis 20%.

Nach einer weiteren bevorzugten Ausführungsform weist das eingesetzte $TiO_2$ die folgende Partikelgrößenverteilung auf: Der $D_{10}$-Wert liegt vorzugsweise bei 0,5 $\mu$m oder darunter; der $D_{50}$-Wert (d.h. der Wert, bei dem jeweils die Hälfte der Partikel einen größeren bzw. kleineren Partikeldurchmesser aufweist) liegt vorzugsweise bei 1,5 $\mu$m oder

darunter; der $D_{90}$-Wert liegt vorzugsweise bei 4 $\mu$m oder darunter. Bevorzugt liegt der $D_{90}$-Wert des eingesetzten $TiO_2$ zwischen etwa 0,5 und 20 $\mu$m, insbesondere zwischen etwa 1 und 10 $\mu$m, besonders bevorzugt zwischen etwa 2 und 5 $\mu$m.

In elektronenmikroskopischen Aufnahmen weist das erfindungsgemäß eingesetzte $TiO_2$ bevorzugt eine offenporige, schwammartige Struktur auf. Die Primärkristallite sind vorzugsweise zu mehr als 30%, insbesondere mehr als 50%, zu vorzugsweise offenporigen Agglomeraten zusammengeschlossen. Es wird angenommen, ohne dass die Erfindung auf diese Annahme beschränkt wäre, dass durch diese besondere Struktur des eingesetzten $TiO_2$, die sich in der Porenradienverteilung spiegelt, besonders günstige Reaktionsbedingungen für die Gasphasenoxidation geschaffen werden.

Nach einer weiteren bevorzugten Ausführungsform enthält die erste Katalysatorlage kein Phosphor. Weiter wird bevorzugt, dass auch die zweite Katalysatorlage kein Phosphor enthält. Besonders bevorzugt enthält nur die letzte Katalysatorlage Phosphor, insbesondere zwischen 0,01 und 0,5 Gew.-%, weiter bevorzugt zwischen 0,1 und 0,4 Gew.-% Phosphor (berechnet als Phosphor).

Je nach vorgesehener Verwendung des erfindungsgemäßen Mehrlagen-Katalysators können neben dem erfindungsgemäß eingesetzten $TiO_2$ die dem Fachmann geläufigen und üblichen Komponenten in der aktiven Masse des Katalysators enthalten sein. Auch die Form des Mehrlagen-Katalysators bzw. dessen homogener oder heterogener Aufbau ist im Sinne der vorliegenden Erfindung grundsätzlich nicht beschränkt und kann jegliche dem Fachmann geläufige und für das jeweilige Anwendungsgebiet geeignet erscheinende Ausführungsform umfassen.

Zur Herstellung von Phthalsäureanhydrid, haben sich insbesondere sogenannte Schalenkatalysatoren bewährt. Hierbei wird ein unter den Reaktionsbedingungen inerter Träger, beispielsweise aus Quarz ($SiO_2$) Porzellan, Magnesiumoxid, Zinndioxid, Siliciumcarbid, Rutil, Tonerde ($Al_2O_3$), Aluminiumsilicat, Magnesiumsilicat (Steatit), Zirkoniumsilicat oder Cersilicat, oder aus Mischungen der vorstehenden Materialien verwendet. Der Träger kann beispielsweise die Form von Ringen, Kugeln, Schalen oder Hohlzylindern aufweisen. Darauf wird in verhältnismäßig dünnen Schichten (Schalen) die katalytisch aktive Masse aufgebracht. Es können auch zwei oder mehrere Schichten der gleichen oder unterschiedlich zusammengesetzter katalytisch aktiver Masse aufgebracht werden.

Bezüglich der weiteren Komponenten der katalytisch aktiven Masse des erfindungsgemäßen Mehrlagen-Katalysators (neben $TiO_2$) kann grundsätzlich auf die im einschlägigen Stand der Technik beschriebenen und dem Fachmann geläufigen Zusammensetzungen bzw. Komponenten verwiesen werden. Dabei handelt es sich hauptsächlich um Katalysatorsysteme, die neben Titanoxid(en) Oxide des Vanadiums enthalten. Solche Katalysatoren sind z.B. in der EP 0 964 744 B1 beschrieben.

Insbesondere sind im Stand der Technik eine Reihe von Promotoren zur Steigerung der Produktivität der Katalysatoren beschrieben, die im erfindungsgemäßen Mehrlagen-Katalysator ebenfalls eingesetzt werden können. Dazu gehören u.a. die Alkali- und Erdalkalimetalle, Thallium, Antimon, Phosphor, Eisen, Niob, Kobalt, Molybdän, Silber, Wolfram, Zinn, Blei und/oder Bismut sowie Mischungen aus zwei oder mehreren der vorstehenden Komponenten. Beispielsweise ist in der DE 21 59 441 A ein Katalysator beschrieben, der neben Titandioxid der Anatas-Modifikation aus 1 bis 30 Gew.-% Vanadiumpentoxid und Zirkondioxid besteht. Über die einzelnen Promotoren lässt sich die Aktivität und Selektivität der Katalysatoren beeinflussen, insbesondere durch Absenkung oder Erhöhung der Aktivität. Zu den die Selektivität erhöhenden Promotoren zählen beispielsweise die Alkalimetalloxide, wohingegen oxidische Phosphorverbindungen, insbesondere Phosphorpentoxid, die Aktivität des Katalysators auf Kosten der Selektivität erhöhen.

Zur Herstellung der erfindungsgemäßen Katalysatoren sind im Stand der Technik zahlreiche geeignete Verfahren beschrieben, so dass eine detaillierte Darstellung hier grundsätzlich nicht erforderlich ist. Zur Herstellung von Schalenkatalysatoren kann beispielsweise auf das in der DE-A-16 42 938 oder der DE-A 17 69 998 beschriebene Verfahren verwiesen werden, worin eine ein wässriges und/oder ein organisches Lösungsmittel enthaltende Lösung oder Suspension der Komponenten der katalytisch aktiven Masse und/oder deren Vorläuferverbindungen (häufig als "Maische" bezeichnet) auf das Trägermaterial in einer beheizten Dragiertrommel bei erhöhter Temperatur aufgesprüht werden, bis der gewünschte Gehalt an katalytisch aktiver Masse, bezogen auf das Katalysatorgesamtgewicht, erreicht ist. Auch lässt sich gemäß der DE 21 06 796 die Aufbringung (Beschichtung) der katalytisch aktiven Masse auf den inerten Träger in Wirbelbeschichtern durchführen.

Bevorzugt werden sogenannte Schalenkatalysatoren durch das Aufbringen einer dünnen Schicht von 50 bis 500 $\mu$m der Aktivkomponenten auf einen inerten Träger hergestellt (z.B. US 2,035,606). Nach einer bevorzugten erfindungsgemäßen Ausführungsform nimmt dabei die Dicke der jeweils aufgebrachten Schicht (mit der Aktivmasse) entsprechend der Abnahme der Aktivmassegehalte wie hierin beschrieben von der ersten zur letzten Katalysatorlage ab. Nimmt die Aktivmasse zwischen den Lagen ab, so nimmt vorzugsweise auch die Schichtdicke der Aktivmasse entsprechend ab. Als Träger haben sich insbesondere Kugeln oder Hohlzylinder bewährt. Diese Formkörper ergeben eine hohe Packungsdichte bei niedrigem Druckverlust und verringern die Gefahr der Bildung von Packungsfehlern beim Einfüllen des Katalysators in die Reaktionsrohre.

Die geschmolzenen und gesinterten Formkörper müssen innerhalb des Temperaturbereiches der ablaufenden Reaktion hitzebeständig sein. Wie vorstehend ausgeführt, kommen dabei beispielsweise Siliciumcarbid, Steatit, Quarz,

Porzellan, SiO$_2$, Al$_2$O$_3$ oder Tonerde in Frage.

Der Vorteil der Beschichtung von Trägerkörpern im Wirbelbett ist die hohe Gleichmäßigkeit der Schichtdicke, die für die katalytische Leistung des Katalysators eine entscheidende Rolle spielt. Eine besonders gleichmäßige Beschichtung erhält man durch Aufsprühen einer Suspension oder Lösung der Aktivkomponenten auf den erwärmten Träger bei 80 bis 200°C im Wirbelbett, beispielsweise gemäß DE 12 80 756, DE 198 28 583 oder DE 197 09 589. Im Gegensatz zu der Beschichtung in Dragiertrommeln kann bei Verwendung von Hohlzylindern als Träger in den genannten Wirbelbettverfahren auch die Innenseite der Hohlzylinder gleichmäßig beschichtet werden. Unter den oben genannten Wirbelbettverfahren ist insbesondere das Verfahren nach DE 197 09 589 von Vorteil, da durch die überwiegend horizontale, kreisförmige Bewegung der Träger neben einer gleichmäßigen Beschichtung auch eine geringe Abrasion von Apparateteilen erreicht wird.

Für den Beschichtungsvorgang wird die wässrige Lösung oder Suspension der Aktivkomponenten und eines organischen Binders, vorzugsweise einem Copolymer aus Vinylacetat/Vinyllaurat, Vinylacetat/Ethylen oder Styrol/Acrylat, über eine oder mehrere Düsen auf den erwärmten, fluidisierten Träger aufgesprüht. Besonders günstig ist es, die Sprühflüssigkeit am Ort der höchsten Produktgeschwindigkeit aufzugeben, wodurch sich der Sprühstoff gleichmäßig im Bett verteilen kann. Der Sprühvorgang wird solange fortgeführt, bis entweder die Suspension verbraucht oder die erforderliche Menge an Aktivkomponenten auf dem Träger aufgebracht ist.

Nach einer besonders bevorzugten erfindungsgemäßen Ausführungsform wird die katalytisch aktive Masse des erfindungsgemäßen Mehrlagen-Katalysators im Fließbett oder Wirbelbett unter Beihilfe geeigneter Bindemittel aufgebracht, so dass ein Schalenkatalysator erzeugt wird. Geeignete Bindemittel umfassen dem Fachmann geläufige organische Binder, bevorzugt Copolymere, vorteilhaft in Form einer wässrigen Dispersion, von Vinylacetat/Vinyllaurat, Vinylacetat/Acrylat, Styrol/Acrylat, Vinylacetat/Maleat sowie Vinylacetat/Ethylen. Besonders bevorzugt wird ein organischer polymerer oder copolymerer Kleber, insbesondere ein Vinylacetat-Copolymer-Kleber, als Bindemittel verwendet. Das verwendete Bindemittel wird in üblichen Mengen der katalytisch aktiven Masse zugegeben, beispielsweise mit etwa 10 bis 20 Gew.-%, bezogen auf den Feststoffgehalt der katalytisch aktiven Masse. Beispielsweise kann auf die EP 744 214 verwiesen werden. Soweit die Aufbringung der katalytisch aktiven Masse bei erhöhten Temperaturen von etwa 150°C erfolgt, ist, wie aus dem Stand der Technik bekannt, eine Aufbringung auf den Träger auch ohne organische Bindemittel möglich. Brauchbare Beschichtungstemperaturen bei Verwendung der vorstehend angegebenen Bindemittel liegen gemäß DE 21 06 796 beispielsweise zwischen etwa 50 und 450°C. Die verwendeten Bindemittel brennen beim Ausheizen des Katalysators bei Inbetriebnahme des gefüllten Reaktors innerhalb kurzer Zeit aus. Die Bindemittel dienen in erster Linie der Verstärkung der Haftung der katalytisch aktiven Masse auf dem Träger und der Verringerung von Abrieb beim Transport und Einfüllen des Katalysators.

Weitere mögliche Verfahren zur Herstellung von Schalenkatalysatoren für die katalytische Gasphasenoxidation von aromatischen Kohlenwasserstoffen zu Carbonsäuren und/oder Carbonsäureanhydriden sind beispielsweise in der WO 98/00778 bzw. EP-A 714 700 beschrieben worden. Danach wird aus einer Lösung und/oder einer Suspension der katalytisch aktiven Metalloxide und/oder deren Vorläuferverbindungen, gegebenenfalls in Anwesenheit von Hilfsmitteln für die Katalysatorherstellung, zunächst ein Pulver hergestellt, das anschließend für die Katalysatorherstellung auf dem Träger, gegebenenfalls nach Konditionierung sowie gegebenenfalls nach Wärmebehandlung zur Erzeugung der katalytisch aktiven Metalloxide schalenförmig aufgebracht und der auf diese Weise beschichtete Träger einer Wärmebehandlung zur Erzeugung der katalytisch aktiven Metalloxide oder einer Behandlung zur Entfernung flüchtiger Bestandteile unterzogen.

Geeignete Bedingungen zur Durchführung eines Verfahrens zur Herstellung von Phthalsäureanhydrid aus o-Xylol und/oder Naphthalin sind dem Fachmann gleichermaßen aus dem Stand der Technik geläufig. Insbesondere wird auf die zusammenfassende Garstellung in K. Towae, W. Enke, R. Jäckh, N. Bhargana "Phtalic Acid and Derivatives" in Ullmann's Encyclopedia of Industrial Chemistry Vol. A. 20, 1992, 181 verwiesen. Beispielsweise können für den stationären Betriebszustand der Oxidation die aus der vorstehenden Literaturstelle der WO-A 98/37967 oder der WO 99/61433 bekannten Randbedingungen gewählt werden.

Dazu werden zunächst die Katalysatoren in die Reaktionsrohre des Reaktors, die von außen auf die Reaktionstemperatur, beispielsweise mittels Salzschmelzen thermostatisiert sind, gefüllt. Über die so bereitete Katalysatorschüttung wird das Reaktionsgas bei Temperaturen von im Allgemeinen 300 bis 450°C, vorzugsweise 320 bis 420°C, und besonders bevorzugt von 340 bis 400°C und bei einem Überdruck von im Allgemeinen 0,1 bis 2,5, vorzugsweise von 0,3 bis 1,5 bar mit einer Raumgeschwindigkeit von im Allgemeinen 750 bis 5000 h$^{-1}$ geleitet.

Das dem Katalysator zugeführte Reaktionsgas wird im Allgemeinen durch Vermischen von einem molekularen Sauerstoff enthaltenden Gas, das außer Sauerstoff noch geeignete Reaktionsmoderatoren und/oder Verdünnungsmittel wie Dampf, Kohlendioxid und/oder Stickstoff enthalten kann, mit dem zu oxidierenden, aromatischen Kohlenwasserstoff erzeugt, wobei das den molekularen Sauerstoff enthaltende Gas im Allgemeinen 1 bis 100, vorzugsweise 2 bis 50 und besonders bevorzugt 10 bis 30 mol-% Sauerstoff, 0 bis 30, vorzugsweise 0 bis 10 mol-% Wasserdampf sowie 0 bis 50, vorzugsweise 0 bis 1 mol-% Kohlendioxid, Rest Stickstoff, enthalten kann. Zur Erzeugung des Reaktionsgases wird das den molekularen Sauerstoff enthaltende Gas im Allgemeinen mit 30 bis 150 g je Nm$^3$ Gas des zu oxidierenden, aromatischen Kohlenwasserstoffs beschickt.

Nach einer besonders bevorzugten erfindungsgemäßen Ausführungsform weist der erfindungsgemäße Mehrlagen-Katalysator einen Aktivmassegehalt zwischen etwa 7 und 12 Gew.-%, bevorzugt zwischen 8 und 10 Gew.-% auf, wobei die Aktivmasse (katalytisch aktive Masse) zwischen 5 bis 15 Gew.-% $V_2O_5$, 0 bis 4 Gew.- $Sb_2O_3$, 0,2 bis 0,75 Gew.-% Cs, 0 bis 3 Gew.-% $Nb_2O_5$ enthält. Neben den vorstehenden Komponenten besteht der Rest der Aktivmasse zu mindestens 90 Gew.-%, vorzugsweise mindestens 95 Gew-%, weiter bevorzugt mindestens 98 Gew-%, insbesondere mindestens 99 Gew-%, weiter bevorzugt mindestens 99,5 Gew-%, insbesondere 100 Gew-% aus $TiO_2$ Ein solcher erfindungsgemäßer Mehrlagen-Katalysator kann beispielsweise vorteilhaft bei einem Zwei- oder Mehrlagen-Katalysator als erste, zur Gaseintrittsseite hin gelegene Katalysatorlage verwendet werden.

Nach einer besonders bevorzugten erfindungsgemäßen Ausführungsform liegt dabei die BET-Oberfläche des Katalysators zwischen 15 und etwa 25 m²/g. Weiterhin wird bevorzugt, dass eine solche erste Katalysatorlage einen Längenanteil von etwa 40 bis 60% an der Gesamtlänge aller vorhandenen Katalysatorlagen (Gesamtlänge des vorhandenen Katalysatorbettes) aufweist.

Nach einer weiteren bevorzugten erfindungsgemäßen Ausführungsform weist der erfindungsgemäße Mehrlagen-Katalysator einen Aktivmassegehalt von etwa 6 bis 11 Gew.-%, insbesondere 7 bis 9 Gew.-% auf, wobei die aktive Masse 5 bis 15 Gew.-% $V_2O_5$, 0 bis 4 Gew.-% $Sb_2O_3$, 0,05 bis 0,3 Gew.-% Cs, 0 bis 2 Gew.-% $Nb_2O_5$ enthält. Neben den vorstehenden Komponenten besteht der Rest der Aktivmasse zu mindestens 90 Gew.-%, vorzugsweise mindestens 95 Gew-%, weiter bevorzugt mindestens 98 Gew-%, insbesondere mindestens 99 Gew-%, weiter bevorzugt mindestens 99,5 Gew-%, insbesondere 100 Gew-% aus $TiO_2$. Ein solcher erfindungsgemäßer Mehrlagen-Katalysator kann beispielsweise vorteilhaft als zweite Katalysatorlage, d.h. stromab der zur Gaseintrittsseite hin gelegenen ersten Katalysatorlage (vgl. oben) eingesetzt werden. Dabei wird bevorzugt, dass der Katalysator eine BET-Oberfläche zwischen etwa 15 und 25 m²/g aufweist. Weiterhin wird bevorzugt, dass diese zweite Lage einen Längenanteil von etwa 10 bis 30% der Gesamtlänge aller vorhandenen Katalysatorlagen einnimmt.

Nach einer weiteren erfindungsgemäßen Ausführungsform weist der erfindungsgemäße Mehrlagen-Katalysator einen Aktivmassegehalt zwischen etwa 5 und 10 Gew.-%, insbesondere zwischen 6 und 8 Gew.-% auf, wobei die Aktivmasse (katalytisch aktive Masse) 5 bis 15 Gew.-% $V_2O_4$, 0 bis 4 Gew.-% $Sb_2O_3$, 0 bis 0,1 Gew.-% Cs, 0 bis 1 Gew.-% $Nb_2O_5$ enthält. Neben den vorstehenden Komponenten besteht der Rest der Aktivmasse zu mindestens 90 Gew.-%, vorzugsweise mindestens 95 Gew-%, weiter bevorzugt mindestens 98 Gew-%, insbesondere mindestens 99 Gew-%, weiter bevorzugt mindestens 99,5 Gew-%, insbesondere 100 Gew-% aus $TiO_2$. Ein solcher erfindungsgemäßer Mehrlagen-Katalysator kann beispielsweise vorteilhaft als dritte, (bzw. letzte) stromab der vorstehend beschriebenen zweiten Katalysatorlage, angeordnete Katalysatorlage eingesetzt werden. Bevorzugt wird dabei eine BET-Oberfläche des Katalysators, die etwas höher liegt als diejenige der näher zur Gaseintrittsseite hin gelegenen Schichten, insbesondere im Bereich zwischen etwa 25 bis etwa 45 m²/g. Weiterhin wird bevorzugt, dass eine solche dritte Katalysatorlage einen Längenanteil von etwa 10 bis 50% der Gesamtlänge aller vorhandenen Katalysatorlagen einnimmt.

Weiterhin wird erfindungsgemäß bevorzugt, dass beim Einsatz des erfindungsgemäßen Mehrlagen-Katalysators in einem Mehrlagen-Katalysatorbett der Gehalt an Alkalimetallen in den Katalysatorlagen von der Gaseintrittsseite zur Gasaustrittsseite hin sinkt. Nach einer besonders bevorzugten Ausführungsform ist der Alkaligehalt, bevorzugt der Cs-Gehalt (berechnet als Cs), in der zweiten Katalysatorlage kleiner als in der ersten Katalysatorlage, und in der dritten Katalysatorlage kleiner als in der zweiten Katalysatorlage (und vorzugsweise ggf. auf die dritte Lage folgenden Lagen). Besonders bevorzugt nimmt somit der Cs-Gehalt (berechnet als Cs) im erfindungsgemäßen Mehrlagen-Katalysator von Lage zu Lage in Gasstromrichtung ab. Nach einer bevorzugten Ausführungsform enthält die dritte (und vorzugsweise auch ggf. nachfolgende Katalysatorlagen) kein Cs. Bevorzugt gilt:

Cs-Gehalt $_{1. Lage}$ > Cs-Gehalt $_{2. Lage}$ >... > Cs-Gehalt $_{letzte Lage}$.

Besonders bevorzugt weist die letzte Katalysatorlage kein Cs auf.

Grundsätzlich kann in dem erfindungsgemäßen Mehrlagen-Katalysator auch ein anderes Titandioxid mit einer anderen Spezifikation als vorstehend beschrieben, d.h. einer anderen BET-Oberfläche, Porosimetrie und/oder Partikelgrößenverteilung, verwendet werden. Erfindungsgemäß wird jedoch besonders bevorzugt, dass mindestens 50%, insbesondere mindestens 75%, besonders bevorzugt das gesamte verwendete $TiO_2$ eine BET-Oberfläche und Porosimetrie wie hierin definiert, und vorzugsweise auch die beschriebene Partikelgrößenverteilung aufweist. Dabei können auch Abmischungen verschiedener $TiO_2$-Materialien verwendet werden.

Auch wurde gefunden, dass nach einer bevorzugten Ausführungsform erfindungsgemäß Mehrlagen-Katalysatoren, die kein Phosphor in der katalytisch aktiven Masse aufweisen, im Zusammenspiel mit dem erfindungsgemäß eingesetzten $TiO_2$ besonders gute Aktivitäten bei gleichzeitig sehr hoher Selektivität ermöglichen. Dabei wird weiterhin bevorzugt, dass mindestens 0,05 Gew.-% der katalytisch aktiven Masse durch mindestens ein Alkalimetall, berechnet als Alkalimetall(e), gebildet wird. Besonders bevorzugt wird als Alkalimetall Cäsium.

Zudem wird nach den Ergebnissen der Erfinder nach einer Ausführungsform bevorzugt, dass der erfindungsgemäße Mehrlagen-Katalysator Niob in einer Menge von 0,01 bis 2 Gew.-%, insbesondere 0,5 bis 1 Gew.-% der katalytisch aktiven Masse enthält.

Die erfindungsgemäßen Mehrlagen-Katalysatoren werden in üblicher Weise vor dem Einsatz temperaturbehandelt bzw. calciniert (konditioniert). Dabei hat sich als vorteilhaft herausgestellt, wenn der Katalysator mindestens 24 Stunden bei mindestens 390°C, insbesondere zwischen 24 und 72 Stunden bei ≥ 400°C, in einem $O_2$-haltigen Gas, insbesondere in Luft, calciniert wird. Die Temperatur sollte vorzugsweise 500°C, insbesondere 470°C, nicht überschreiten. Grundsätzlich sind jedoch auch andere Calcinierungsbedingungen, die dem Fachmann als geeignet erscheinen, nicht ausgeschlossen.

Ein Verfahren zur Herstellung eines Mehrlagen-Katalysators gemäß einem der vorstehenden Ansprüche, umfasst die folgenden Schritte:

a. Bereitstellen einer katalytisch aktiven Masse wie hierin definiert,

b. Bereitstellen eines inerten Trägers, insbesondere eines inerten Trägerformkörpers;

c. Aufbringen der katalytisch aktiven Masse auf den inerten Träger, insbesondere in einer Wirbelschicht oder einem Fließbett.

Nach einem weiteren Aspekt betrifft die Erfindung auch ein Verfahren zur Herstellung von Phthalsäureanhydrid durch Gasphasenoxidation von o-Xylol und/oder Naphthalin, wobei ein drei- oder mehrschichtiger Katalysator wie in der vorliegenden Beschreibung definiert verwendet wird.

Nach einem weiteren Aspekt betrifft die vorliegende Erfindung schließlich auch die Verwendung eines Mehrlagen-Katalysators wie hierin definiert zur Herstellung von Phthalsäureanhydrid durch Gasphasenoxidation von o-Xylol und/oder Naphthalin.

## METHODEN

Zur Bestimmung der Parameter der erfindungsgemäßen Katalysatoren werden die nachstehenden Methoden eingesetzt:

1. BET-Oberfläche:

Die Bestimmung erfolgt nach der BET-Methode gemäß DIN 66131; eine Veröffentlichung der BET-Methode findet sich auch in J. Am. Chem. Soc. 60, 309 (1938).

2. Porenradienverteilung:

Die Bestimmung der Porenradienverteilung und des Porenvolumens des eingesetzten $TiO_2$ erfolgte mittels Quecksilberporosimetrie gemäß DIN 66133; maximaler Druck: 2.000 bar, Porosimeter 4000 (Firma Porotec, DE), nach Angaben des Herstellers.

3. Primärkristallitgrößen:

Die Bestimmung der Primärkristallitgrößen erfolgte mittels Pulver-Röntgendiffraktometrie. Die Analyse wurde mit einem Gerät der Firma Bruker, DE, durchgeführt: Typ BRUKER AXS - D4 Endeavor. Die erhaltenen Röntgendiffraktogramme wurden mit dem Softwarepaket "DiffracPlus D4 Measurement" gemäß den Angaben des Herstellers aufgezeichnet und die Halbwertsbreite des 100% Reflexes wurde mit der Software "DiffracPlus Evaluation" nach der Debye-Scherrer Formel gemäß den Angaben des Herstellers ausgewertet, um die Primärkristallitgröße zu bestimmen.

4. Partikelgrößen:

Die Bestimmung der Partikelgrößen erfolgte nach der Laserbeugungsmethode mit einem Fritsch Particle Sizer Analysette 22 Economy (Fa. Fritsch, DE) nach den Angaben des Herstellers, auch bezüglich der Probenvorbehandlung: die Probe wird in deionisiertem Wasser ohne Zusatz von Hilfsmitteln homogenisiert und 5 Minuten mit Ultraschall behandelt.

5. Alkaligehalt des $TiO_2$

Der Alkaligehalt des $TiO_2$ wird nach DIN ISO 9964-3 bestimmt. So kann eine Alkalibestimmung mittels ICP-AES (Inductively Coupled Plasma Atomic Emission Spectroscopy) erfolgen und ggf. der Gesamtalkaligehalt des $TiO_2$ aufaddiert werden.

6. Schüttdichte:

Die Schüttdichte wurde anhand des zur Herstellung des Katalysators eingesetzten $TiO_2$ (bei 150°C im Vakuum getrocknet, uncalciniert) bestimmt. Die erhaltenen Werte aus drei Bestimmungen wurden gemittelt.

Die Schüttdichte wurde bestimmt, indem 100 g des $TiO_2$-Materials in eine 1.000 ml-Dose eingefüllt und ca. 30 Sekunden geschüttelt wurden (im Bedarfsfall mehrere parallele Ansätze).

Ein Messzylinder (Fassungsvermögen genau 100ml) wird leer auf 10 mg gewogen. Darauf wird der Pulvertrichter mit Stativ und Klemme über die Öffnung des Zylinders befestigt. Nach Ingangsetzung der Stoppuhr wird der Messzylinder innerhalb von 15 Sekunden mit dem $TiO_2$-Material gefüllt. Mit dem Spatel wird laufend Füllgut nachgeschüttet, so dass der Messzylinder immer leicht überstehend gefüllt ist. Nach 2 Minuten wird mit dem Spatel der Überstand abgestreift, wobei darauf zu achten ist, dass keine Presskräfte das Material im Zylinder verdichten. Der gefüllte Messzylinder wird abgepinselt und gewogen.

Die Schüttdichte wird in g/ml angegeben.

Die Bestimmung der BET-Oberfläche, der Porenradienverteilung bzw. des Porenvolumens sowie der Partikelgrößenverteilung erfolgte bezüglich des Titandioxids jeweils an dem bei 150°C im Vakuum getrockneten, uncalcinierten Material.

Auch die Angaben in der vorliegenden Beschreibung bezüglich der BET-Oberflächen der Katalysatoren bzw. Katalysatorlagen beziehen sich auf die BET-Oberflächen des jeweils eingesetzten $TiO_2$-Material (getrocknet in Vakuum bei 150°C, uncalciniert, vgl. oben).

In der Regel wird die BET-Oberfläche des Katalysators durch die BET-Oberfläche des eingesetzten $TiO_2$ bestimmt, wobei durch den Zusatz weiterer katalytisch aktiver Komponenten die BET-Oberfläche in gewissem Umfang verändert wird. Dies ist dem Fachmann geläufig.

Der Aktivmasseanteil (Anteil der katalytisch aktiven Masse, ohne Bindemittel) bezieht sich jeweils auf den Anteil (in Gew.-%) der katalytisch aktiven Masse an dem Gesamtgewicht des Katalysators einschließlich Träger in der jeweiligen Katalysatorlage, gemessen nach Konditionierung über 4h bei 400°C.

Die Erfindung wird nun anhand der nachstehenden, nicht beschränkenden Beispiele näher erläutert:

## BEISPIELE

### Beispiel 1: Herstellung von Katalysator A

Zur Herstellung des Katalysators A mit einem Aktivmassenanteil von 8 Gew.-% und der Zusammensetzung von 7,5 Gew.-% Vanadiumpentoxid, 3,2 Gew.-% Antimontrioxid, 0,40 Gew.-% Cäsium (berechnet als Cäsium), 0,2 Gew.-% Phosphor (berechnet als Phosphor) und Rest Titandioxid wurden in einem sogenannten Wirbelbett-Coater 2600 g Steatitkörper in Form von Hohlzylindern der Größe 8 x 6 x 5 mm mit einer Suspension aus 17,9 g Vanadiumpentoxid, 7,6 g Antimontrioxid, 1,3 g Cäsiumsulfat, 1,9 g Ammoniumdihydrogenphosphat, 211,1 g Titandioxid mit einer BET-Oberfläche von 21 $m^2$/g, 130,5 g Bindemittel aus einer 50%-igen Dispersion von Wasser und Vinylacetat/Ethylencopolymer (Vinnapas® EP 65 W, Fa. Wacker) und 2000 g Wasser bei einer Temperatur von 70°C beschichtet. Die aktive Masse wurde in Form dünner Schichten aufgetragen.

### Beispiel 2: Herstellung von Katalysator B

Zur Herstellung des Katalysators B mit einem Aktivmassenanteil von 8 Gew.-% und der Zusammensetzung von 7,5 Gew.-% Vanadiumpentoxid, 3,2 Gew.-% Antimontrioxid, 0,20 Gew.-% Cäsium (berechnet als Cäsium), 0,2 Gew.-% Phosphor (berechnet als Phosphor) und Rest Titandioxid wurden in einem sogenannten Wirbelbett-Coater 2200 g Steatitkörper in Form von Hohlzylindern der Größe 8 x 6 x 5 mm mit einer Suspension aus 15,4 g Vanadiumpentoxid, 6,6 g Antimontrioxid, 0,5 g Cäsiumcarbonat, 1,5 g Ammoniumdihydrogenphosphat, 182,9 g Titandioxid mit einer BET-Oberfläche von 21 $m^2$/g, 110,7 g Bindemittel aus einer 50%-igen Dispersion von Wasser und Vinylacetat/Ethylencopolymer (Vinnapas® EP 65 W, Fa. Wacker) und 2000 g Wasser bei einer Temperatur von 70°C beschichtet. Die aktive Masse wurde in Form dünner Schichten aufgetragen.

Beispiel 3: Herstellung von Katalysator C

Zur Herstellung des Katalysators C mit einem Aktivmassenanteil von 8 Gew.-% und der Zusammensetzung von 7,5 Gew.-% Vanadiumpentoxid, 3,2 Gew.-% Antimontrioxid, 0,2 Gew.-% Phosphor (berechnet als Phosphor) und Rest Titandioxid wurden in einem sogenannten Wirbelbett-Coater 2200 g Steatitkörper in Form von Hohlzylindern der Größe 8 x 6 x 5 mm mit einer Suspension aus 13,35 g Vanadiumpentoxid, 5,7 g Antimontrioxid, 1,34 g Ammoniumdihydrogenphosphat, 158,65 g Titandioxid mit einer BET-Oberfläche von 21 m$^2$/g, 109,4 g Bindemittel aus einer 50%-igen Dispersion von Wasser und Vinylacetat/Ethylencopolymer (Vinnapas® EP 65 W, Fa. Wacker) und 2000 g Wasser bei einer Temperatur von 70°C beschichtet. Die aktive Masse wurde in Form dünner Schichten aufgetragen.

Beispiel 4: Herstellung von Katalysator D

Zur Herstellung des Katalysators D mit einem Aktivmassenanteil von 9 Gew.-% und der Zusammensetzung von 7,5 Gew.-% Vanadiumpentoxid, 3,2 Gew.-% Antimontrioxid, 0,40 Gew.-% Cäsium (berechnet als Cäsium), 0,2 Gew.-% Phosphor (berechnet als Phosphor) und Rest Titandioxid wurden in einem sogenannten Wirbelbett-Coater 2000 g Steatitkörper in Form von Hohlzylindern der Größe 8 x 6 x 5 mm mit einer Suspension aus 17,0 g Vanadiumpentoxid, 7,0 g Antimontrioxid, 1,1 g Cäsiumsulfat, 1,65 g Ammoniumdihydrogenphosphat, 194,9 g Titandioxid mit einer BET-Oberfläche von 21 m$^2$/g, 102,1 g Bindemittel aus einer 50%-igen Dispersion von Wasser und Vinylacetat/Ethylencopolymer (Vinnapas® EP 65 W, Fa. Wacker) und 2000 g Wasser bei einer Temperatur von 70°C beschichtet. Die aktive Masse wurde in Form dünner Schichten aufgetragen.

Beispiel 5: Herstellung von Katalysator E

Zur Herstellung des Katalysators E mit einem Aktivmassenanteil von 8 Gew.-% und der Zusammensetzung von 7,5 Gew.-% Vanadiumpentoxid, 3,2 Gew.-% Antimontrioxid, 0,20 Gew.-% Cäsium (berechnet als Cäsium), 0,2 Gew.-% Phosphor (berechnet als Phosphor) und Rest Titandioxid wurden in einem sogenannten Wirbelbett-Coater 2000 g Steatitkörper in Form von Hohlzylindern der Größe 8 x 6 x 5 mm mit einer Suspension aus 15,1 g Vanadiumpentoxid, 6,3 g Antimontrioxid, 0,53 g Cäsiumsulfat, 1,47 g Ammoniumdihydrogenphosphat, 173,7 g Titandioxid mit einer BET-Oberfläche von 21 m$^2$/g, 101 g Bindemittel aus einer 50%-igen Dispersion von Wasser und Vinylacetat/ Ethylencopolymer (Vinnapas® EP 65 W, Fa. Wacker) und 2000 g Wasser bei einer Temperatur von 70°C beschichtet. Die aktive Masse wurde in Form dünner Schichten aufgetragen.

Beispiel 6: Herstellung von Katalysator F

Zur Herstellung des Katalysators F mit einem Aktivmassenanteil von 8 Gew.-% und der Zusammensetzung von 7,5 Gew.-% Vanadiumpentoxid, 3,2 Gew.-% Antimontrioxid, 0,2 Gew.-% Phosphor (berechnet als Phosphor) und Rest Titandioxid wurden in einem sogenannten Wirbelbett-Coater 2000 g Steatitkörper in Form von Hohlzylindern der Größe 8 x 6 x 5 mm mit einer Suspension aus 15,1 g Vanadiumpentoxid, 6,25 g Antimontrioxid, 1,47 g Ammoniumdihydrogenphosphat, 174,11 g Titandioxid mit einer BET-Oberfläche von 27 m$^2$/g, 101 g Bindemittel aus einer 50%-igen Dispersion von Wasser und Vinylacetat/Ethylencopolymer (Vinnapas® EP 65 W, Fa. Wacker) und 2000 g Wasser bei einer Temperatur von 70°C beschichtet. Die aktive Masse wurde in Form dünner Schichten aufgetragen.

Beispiel 7: Herstellung von Katalysator G

Zur Herstellung des Katalysators G mit einem Aktivmassenanteil von 8 Gew.-% und der Zusammensetzung von 7,5 Gew.-% Vanadiumpentoxid, 3,2 Gew.-% Antimontrioxid, 0,2 Gew.-% Phosphor (berechnet als Phosphor) und Rest Titandioxid wurde genau wie vorstehend in Beispiel 6 bzgl. Katalysator F vorgegangen, wobei jedoch Titandioxid mit einer BET-Oberfläche von 21 m$^2$/g eingesetzt wurde.

Beispiel 8: Katalytische Leistungsdaten bei der Oxidation von o-Xylol zu Phthalsäureanhydrid (Vergleichsbeispiel 1)

In ein 450 cm langes Reaktionsrohr werden hintereinander 100 cm des Katalysators C, 60 cm des Katalysators B und 130 cm des Katalysators A gefüllt. Das Reaktionsrohr befindet sich in einer flüssigen Salzschmelze, die auf Temperaturen bis 450°C aufgeheizt werden kann. In der Katalysatorschüttung befindet sich ein 3 mm Schutzrohr mit eingebauten Thermoelement, über das die Katalysatortemperatur über die komplette Katalysatorkombination angezeigt werden kann. Zur Ermittlung der katalytischen Leistungsdaten werden über diese Katalysatorkombination in der Reihenfolge ABC von 0 bis maximal 70 g/Nm$^3$ o-Xylol (Reinheit 99,9%) bei 3,6 Nm$^3$ Luft/h geleitet und das Reaktionsgas nach Reaktionsrohraustritt durch einen Kondensator geleitet, indem sich alle organischen Bestandteile des Reaktionsgases, bis auf das Kohlenmonoxid und Kohlendioxid, abscheiden. Das abgeschiedene Rohprodukt wird mittels überhitzten Dampf abgeschmolzen, aufgefangen und anschließend verwogen.

Die Rohausbeute wird wie folgt bestimmt.

$$\textit{Max. Roh-PSA-Ausbeute [Gew.-\%]}$$

$$=$$

$$\textit{Ausgewogene Menge Roh-PSA [g] x 100 / Zulauf o-Xylol [g] x Reinheit o-Xylol [\%/100]}$$

In einem weiteren Versuch (Beispiel 8a) wurden die Lagenlängen wie folgt variiert: 90 cm Katalysator C, 60 cm Katalysator B, 140 cm Katalysator A. Ansonsten wurde wie in Beispiel 8 vorgegangen.

Die Ergebnisse des Testlaufs sind in der Tabelle 1 aufgeführt.

Beispiel 9: Katalytische Leistungsdaten bei der Oxidation von o-Xylol zu Phthalsäureanhydrid (Erfindungsgemäßes Beispiel 1)

In ein 450 cm langes Reaktionsrohr werden hintereinander 90 cm des Katalysators F, 60 cm des Katalysators E und 140 cm des Katalysators D gefüllt. Ansonsten geht man wie unter Beispiel 8 beschrieben vor. Die Ergebnisse des Testlaufs sind in der Tabelle 1 aufgeführt.

Beispiel 10 : Katalytische Leistungsdaten bei der Oxidation von o-Xylol zu Phthalsäureanhydrid (Vergleichsbeispiel 2)

In ein 450 cm langes Reaktionsrohr werden hintereinander 130 cm des Katalysators C, 60 cm des Katalysators B und 100 cm des Katalysators A gefüllt. Ansonsten geht man wie unter Beispiel 8 beschrieben vor. Die Ergebnisse des Testlaufs sind in der Tabelle 1 aufgeführt.

Beispiel 11 : Katalytische Leistungsdaten bei der Oxidation von o-Xylol zu Phthalsäureanhydrid (Erfindungsgemäßes Beispiel 2)

In ein 450 cm langes Reaktionsrohr werden hintereinander 90 cm des Katalysators G, 60 cm des Katalysators E und 140 cm des Katalysators D gefüllt. Ansonsten geht man wie unter Beispiel 8 beschrieben vor. Die Ergebnisse des Testlaufs sind in der Tabelle 1 aufgeführt.

Tabelle 1

| Beispiel | Maximal Beladung | Roh-PSA-Ausbeute | PSA-Qualität (Phthalidwert im Reaktionsgas) | Hotspottemperatur undlage |
|---|---|---|---|---|
| Beispiel 8: Katalysatorkombination A (130 cm) B (60 cm) C (100 cm) | 50 g/Nm$^3$ | 112,4 Gew.-% | > 2000 ppm | 450 °C 150 cm (2. Lage) |
| Beispiel 8a: Katalysatorkombination A (140 cm) B (60 cm) C (90 cm) | 54 g/Nm$^3$ | 112,6 Gew.-% | >2200 ppm | 447 °C 150 cm (2. Lage) |
| Beispiel 9: Katalysatorkombination D (140 cm) E (60 cm) F (90 cm) | 57 g/Nm$^3$ | 113,8 Gew.-% | < 500 ppm | 440 °C 50 cm (1. Lage) |

(fortgesetzt)

| Beispiel | Maximal Beladung | Roh-PSA-Ausbeute | PSA-Qualität (Phthalidwert im Reaktionsgas) | Hotspottemperatur undlage |
|---|---|---|---|---|
| Beispiel 10: Katalysatorkombination A (100 cm) B (60 cm) C (130 cm) | 45 g/Nm$^3$ | 106,7 Gew.-% | > 10000 ppm | 450°C 150 cm (2. Lage) |
| Beispiel 11: Katalysatorkombination D (140 cm) E (60 cm) G (90 cm) | 58 g/Nm$^3$ | 113,6 Gew.-% | < 800 ppm | 440 °C 50 cm (1. Lage) |

Wie aus Tabelle 1 ersichtlich, zeigen die erfindungsgemäßen Katalysatoren gemäß der Beispiele 9 und 11 die höchste PSA-Ausbeute und höchste PSA-Qualität. Der Hot Spot ist vorteilhaft in der ersten Katalysatorlage positioniert. Dabei ist das erfindungsgemäße Beispiel 9, bei dem die BET-Oberfläche von der ersten zur dritten Katalysatorlage ansteigt (hier: in der dritten Katalysatorlage höher ist als in der ersten und zweiten Katalysatorlage), noch besser bezüglich der PSA-Qualität als das erfindungsgemäße Beispiel 11, bei dem die BET-Oberfläche nicht von der ersten zur dritten Katalysatorlage hin ansteigt.

Bei den folgenden Beispielen wurde als einziges Merkmal der Aktivmassengehalt in den einzelnen Lagen geändert.

Beispiel 12 (Vergleich)

Eingesetzte Katalysatoren:

Lage 1: Katalysator H, 9 Gew.-% Aktivmassengehalt
Lage 2: Katalysator I, 10 Gew.-% Aktivmassengehalt
Lage 3: Katalysator J, 11 Gew.-% Aktivmassengehalt)

Zur Herstellung des Katalysators H mit einem Aktivmassenanteil von 9 Gew.-% und der Zusammensetzung von 7,5 Gew.-% Vanadiumpentoxid, 3,2 Gew.-% Antimontrioxid, 0,40 Gew.-% Cäsium (berechnet als Cäsium), 0,2 Gew.-% Phosphor (berechnet als Phosphor) und Rest Titandioxid wurden in einem sogenannten Wirbelbett-Coater 2600 g Steatitkörper in Form von Hohlzylindern der Größe 8 x 6 x 5 mm mit einer Suspension aus 20,3 g Vanadiumpentoxid, 8,7 g Antimontrioxid, 1,5 g Cäsiumsulfat, 2,0 g Ammoniumdihydrogenphosphat, 240,1 g Titandioxid mit einer BET-Oberfläche von 21 m$^2$/g, 132,1 g Bindemittel aus einer 50%-igen Dispersion von Wasser und Vinylacetat/Ethylencopolymer (Vinnapas® EP 65 W, Fa. Wacker) und 2000 g Wasser bei einer Temperatur von 70°C beschichtet. Die aktive Masse wurde in Form dünner Schichten aufgetragen.

Zur Herstellung des Katalysators I mit einem Aktivmassenanteil von 10 Gew.-% und der Zusammensetzung von 7,5 Gew.-% Vanadiumpentoxid, 3,2 Gew.-% Antimontrioxid, 0,20 Gew.-% Cäsium (berechnet als Cäsium), 0,2 Gew.-% Phosphor (berechnet als Phosphor) und Rest Titandioxid wurden in einem sogenannten Wirbelbett-Coater 2600 g Steatitkörper in Form von Hohlzylindern der Größe 8 x 6 x 5 mm mit einer Suspension aus 22,8 g Vanadiumpentoxid, 9,7 g Antimontrioxid, 0,8 g Cäsiumsulfat, 2,3 g Ammoniumdihydrogenphosphat, 270,3 g Titandioxid mit einer BET-Oberfläche von 21 m$^2$/g, 133,6 g Bindemittel aus einer 50%-igen Dispersion von Wasser und Vinylacetat/Ethylencopolymer (Vinnapas® EP 65 W, Fa. Wacker) und 2300 g Wasser bei einer Temperatur von 70°C beschichtet. Die aktive Masse wurde in Form dünner Schichten aufgetragen.

Zur Herstellung des Katalysators J mit einem Aktivmassenanteil von 11 Gew.-% und der Zusammensetzung von 7,5 Gew.-% Vanadiumpentoxid, 3,2 Gew.-% Antimontrioxid, 0,2 Gew.-% Phosphor (berechnet als Phosphor) und Rest Titandioxid wurden in einem sogenannten Wirbelbett-Coater 2600 g Steatitkörper in Form von Hohlzylindern der Größe 8 x 6 x 5 mm mit einer Suspension aus 25,4 g Vanadiumpentoxid, 10,8 g Antimontrioxid, 2,5 g Ammoniumdihydrogenphosphat, 301,4 g Titandioxid mit einer BET-Oberfläche von 27 m$^2$/g, 135,2 g Bindemittel aus einer 50%-igen Dispersion von Wasser und Vinylacetat/Ethylencopolymer (Vinnapas® EP 65 W, Fa. Wacker) und 2500 g Wasser bei einer Temperatur von 70°C beschichtet. Die aktive Masse wurde in Form dünner Schichten aufgetragen.

Beispiel 13 (erfindungsgemäß)

Eingesetzte Katalysatoren:

Lage 1: Katalysator K, 9 Gew.-% Aktivmasengehalt
Lage 2: Katalysator L, 8 Gew.-% Aktivmassengehalt
Lage 3: Katalysator M, 7 Gew.-% Aktivmassengehalt

Zur Herstellung des Katalysators K mit einem Aktivmassenanteil von 9 Gew.-% und der Zusammensetzung von 7,5 Gew.-% Vanadiumpentoxid, 3,2 Gew.-% Antimontrioxid, 0,40 Gew.-% Cäsium (berechnet als Cäsium), 0,2 Gew.-% Phosphor (berechnet als Phosphor) und Rest Titandioxid wurden in einem sogenannten Wirbelbett-Coater 2600 g Steatitkörper in Form von Hohlzylindern der Größe 8 x 6 x 5 mm mit einer Suspension aus 20,3 g Vanadiumpentoxid, 8,7 g Antimontrioxid, 1,5 g Cäsiumsulfat, 2,0 g Ammoniumdihydrogenphosphat, 240,1 g Titandioxid mit einer BET-Oberfläche von 21 $m^2$/g, 132,1 g Bindemittel aus einer 50%-igen Dispersion von Wasser und Vinylacetat/Ethylencopolymer (Vinnapas® EP 65 W, Fa. Wacker) und 2000 g Wasser bei einer Temperatur von 70°C beschichtet. Die aktive Masse wurde in Form dünner Schichten aufgetragen.

Zur Herstellung des Katalysators L mit einem Aktivmassenanteil von 8 Gew.-% und der Zusammensetzung von 7,5 Gew.-% Vanadiumpentoxid, 3,2 Gew.-% Antimontrioxid, 0,20 Gew.-% Cäsium (berechnet als Cäsium), 0,2 Gew.-% Phosphor (berechnet als Phosphor) und Rest Titandioxid wurden in einem sogenannten Wirbelbett-Coater 2600 g Steatitkörper in Form von Hohlzylindern der Größe 8 x 6 x 5 mm mit einer Suspension aus 17,9 g Vanadiumpentoxid, 7,6 g Antimontrioxid, 0,6 g Cäsiumsulfat, 1,8 g Ammoniumdihydrogenphosphat, 211,6 g Titandioxid mit einer BET-Oberfläche von 21 $m^2$/g, 132,1 g Bindemittel aus einer 50%-igen Dispersion von Wasser und Vinylacetat/Ethylencopolymer (Vinnapas® EP 65 W, Fa. Wacker) und 2000 g Wasser bei einer Temperatur von 70°C beschichtet. Die aktive Masse wurde in Form dünner Schichten aufgetragen.

Zur Herstellung des Katalysators M mit einem Aktivmassenanteil von 7 Gew.-% und der Zusammensetzung von 7,5 Gew.-% Vanadiumpentoxid, 3,2 Gew.-% Antimontrioxid, 0,2 Gew.-% Phosphor (berechnet als Phosphor) und Rest Titandioxid wurden in einem sogenannten Wirbelbett-Coater 2600 g Steatitkörper in Form von Hohlzylindern der Größe 8 x 6 x 5 mm mit einer Suspension aus 15,5 g Vanadiumpentoxid, 6,6 g Antimontrioxid, 1,6 g Ammoniumdihydrogenphosphat, 183,5 g Titandioxid mit einer BET-Oberfläche von 27 $m^2$/g, 129,1 g Bindemittel aus einer 50%-igen Dispersion von Wasser und Vinylacetat/Ethylencopolymer (Vinnapas® EP 65 W, Fa. Wacker) und 1800 g Wasser bei einer Temperatur von 70°C beschichtet. Die aktive Masse wurde in Form dünner Schichten aufgetragen.

Die 3-Lagen-Katalysatoren der Beispiele 12 und 13 wurden wie folgt eingesetzt:
In ein 450 cm langes Reaktionsrohr werden 150 cm des Katalysators H (Beispiel 12) bzw. K (Beispiel 13), 60 cm des Katalysators I (Beispiel 12) bzw. L (Beispiel 13) und 80 cm des Katalysators J (Beispiel 12) bzw. M (Beispiel 13) gefüllt. Ansonsten geht man wie unter Beispiel 8 beschrieben vor. Die Ergebnisse des Testlaufs sind in der Tabelle 2 aufgeführt.

Tabelle 2

| • | | | |
|---|---|---|---|
| **Beispiel** | **Maximal Beladung** | **Roh-PSA-Ausbeute** | **Hotspottemperatur undlage** |
| 12 (Vergleich): Katalysatorkombination H (150 cm), I (60 cm) J (80 cm) | 60 g/Nm$^3$ | 109,4 Gew.-% | 448 °C 55 cm (1. Lage) |
| 13 (Efindungsgemäß): Katalysatorkombination K (150 cm) L (60 cm) M (80 cm) | 60 g/Nm$^3$ | 114,1 Gew.-% | 450 °C 55 cm (1. Lage) |

Wie aus Tabelle 2 ersichtlich, zeigt der erfindungsgemäße 3-Lagen-Katalysator gemäß Beispiel 13 eine erheblich bessere PSA-Ausbeute.

Beispiel 14 (Vergleich)

Eingesetzte Katalysatoren:

Lage 1: Katalysator N, 8 Gew.-% Aktivmassengehalt
Lage 2: Katalysator O, 9 Gew.-% Aktivmassengehalt

Lage 3: Katalysator P, 10 Gew.-% Aktivmassengehalt)

Zur Herstellung des Katalysators N mit einem Aktivmassenanteil von 8 Gew.-% und der Zusammensetzung von 7,5 Gew.-% Vanadiumpentoxid, 3,2 Gew.-% Antimontrioxid, 0,40 Gew.-% Cäsium (berechnet als Cäsium) und Rest Titandioxid wurden in einem sogenannten Wirbelbett-Coater 2600 g Steatitkörper in Form von Hohlzylindern der Größe 8 x 6 x 5 mm mit einer Suspension aus 17,9 g Vanadiumpentoxid, 7,6 g Antimontrioxid, 1,3 g Cäsiumsulfat, 211,6 g Titandioxid mit einer BET-Oberfläche von 21 $m^2$/g, 130,5 g Bindemittel aus einer 50%-igen Dispersion von Wasser und Vinylacetat/Ethylencopolymer (Vinnapas® EP 65 W, Fa. Wacker) und 2000 g Wasser bei einer Temperatur von 70°C beschichtet. Die aktive Masse wurde in Form dünner Schichten aufgetragen.

Zur Herstellung des Katalysators O mit einem Aktivmassenanteil von 9 Gew.-% und der Zusammensetzung von 7,5 Gew.-% Vanadiumpentoxid, 3,2 Gew.-% Antimontrioxid, 0,20 Gew.-% Cäsium (berechnet als Cäsium) und Rest Titandioxid wurden in einem sogenannten Wirbelbett-Coater 2600 g Steatitkörper in Form von Hohlzylindern der Größe 8 x 6 x 5 mm mit einer Suspension aus 20,3 g Vanadiumpentoxid, 8,7 g Antimontrioxid, 0,7 g Cäsiumsulfat, 241,2 g Titandioxid mit einer BET-Oberfläche von 21 $m^2$/g, 132,1 g Bindemittel aus einer 50%-igen Dispersion von Wasser und Vinylacetat/Ethylencopolymer (Vinnapas® EP 65 W, Fa. Wacker) und 2300 g Wasser bei einer Temperatur von 70°C beschichtet. Die aktive Masse wurde in Form dünner Schichten aufgetragen.

Zur Herstellung des Katalysators P mit einem Aktivmassenanteil von 10 Gew.-% und der Zusammensetzung von 7,5 Gew.-% Vanadiumpentoxid, 0,2 Gew.-% Phosphor (berechnet als Phosphor) und Rest Titandioxid wurden in einem sogenannten Wirbelbett-Coater 2600 g Steatitkörper in Form von Hohlzylindern der Größe 8 x 6 x 5 mm mit einer Suspension aus 22,8 g Vanadiumpentoxid, 2,3 g Ammoniumdihydrogenphosphat, 280,7 g Titandioxid mit einer BET-Oberfläche von 27 $m^2$/g, 133,6 g Bindemittel aus einer 50%-igen Dispersion von Wasser und Vinylacetat/Ethylencopolymer (Vinnapas® EP 65 W, Fa. Wacker) und 2500 g Wasser bei einer Temperatur von 70°C beschichtet. Die aktive Masse wurde in Form dünner Schichten aufgetragen.

Beispiel 15 (erfindungsgemäß)

Eingesetzte Katalysatoren:

Lage 1: Katalysator Q, 8 Gew.-% Aktivmasengehalt
Lage 2: Katalysator R, 8 Gew.-% Aktivmassengehalt
Lage 3: Katalysator S, 7 Gew.-% Aktivmassengehalt

Zur Herstellung des Katalysators Q mit einem Aktivmassenanteil von 8 Gew.-% und der Zusammensetzung von 7,5 Gew.-% Vanadiumpentoxid, 3,2 Gew.-% Antimontrioxid, 0,40 Gew.-% Cäsium (berechnet als Cäsium), und Rest Titandioxid wurden in einem sogenannten Wirbelbett-Coater 2600 g Steatitkörper in Form von Hohlzylindern der Größe 8 x 6 x 5 mm mit einer Suspension aus 17,9 g Vanadiumpentoxid, 7,6 g Antimontrioxid, 1,3 g Cäsiumsulfat, 211,6 g Titandioxid mit einer BET-Oberfläche von 21 $m^2$/g, 130,5 g Bindemittel aus einer 50%-igen Dispersion von Wasser und Vinylacetat/Ethylencopolymer (Vinnapas® EP 65 W, Fa. Wacker) und 2000 g Wasser bei einer Temperatur von 70°C beschichtet. Die aktive Masse wurde in Form dünner Schichten aufgetragen.

Zur Herstellung des Katalysators R mit einem Aktivmassenanteil von 8 Gew.-% und der Zusammensetzung von 7,5 Gew.-% Vanadiumpentoxid, 3,2 Gew.-% Antimontrioxid, 0,20 Gew.-% Cäsium (berechnet als Cäsium), und Rest Titandioxid wurden in einem sogenannten Wirbelbett-Coater 2600 g Steatitkörper in Form von Hohlzylindern der Größe 8 x 6 x 5 mm mit einer Suspension aus 17,9 g Vanadiumpentoxid, 7,6 g Antimontrioxid, 0,6 g Cäsiumsulfat, 212,1 g Titandioxid mit einer BET-Oberfläche von 21 $m^2$/g, 130,5 g Bindemittel aus einer 50%-igen Dispersion von Wasser und Vinylacetat/Ethylencopolymer (Vinnapas® EP 65 W, Fa. Wacker) und 2000 g Wasser bei einer Temperatur von 70°C beschichtet. Die aktive Masse wurde in Form dünner Schichten aufgetragen.

Zur Herstellung des Katalysators S mit einem Aktivmassenanteil von 7 Gew.-% und der Zusammensetzung von 7,5 Gew.-% Vanadiumpentoxid, 0,2 Gew.-% Phosphor (berechnet als Phosphor) und Rest Titandioxid wurden in einem sogenannten Wirbelbett-Coater 2600 g Steatitkörper in Form von Hohlzylindern der Größe 8 x 6 x 5 mm mit einer Suspension aus 15,5 g Vanadiumpentoxid, 1,6 g Ammoniumdihydrogenphosphat, 190,1 g Titandioxid mit einer BET-Oberfläche von 27 $m^2$/g, 129,1 g Bindemittel aus einer 50%-igen Dispersion von Wasser und Vinylacetat/Ethylencopolymer (Vinnapas® EP 65 W, Fa. Wacker) und 1800 g Wasser bei einer Temperatur von 70°C beschichtet. Die aktive Masse wurde in Form dünner Schichten aufgetragen.

Die 3-Lagen-Katalysatoren der Beispiele 14 und 15 wurden wie folgt eingesetzt:
In ein 450 cm langes Reaktionsrohr werden hintereinander 160 cm des Katalysators N (Beispiel 14) bzw. Q (Beispiel 15), 60 cm des Katalysators O (Beispiel 14) bzw. R (Beispiel 15)und 70 cm des Katalysators P (Beispiel 14) bzw. S (Beispiel 15) gefüllt. Ansonsten geht man wie unter Beispiel 8 beschrieben vor. Die Ergebnisse des Testlaufs sind in der Tabelle 3 aufgeführt.

Tabelle 3

| Beispiel | Maximal Beladung | Roh-PSA-Ausbeute | Hotspottemperatur undlage |
|---|---|---|---|
| 14 (Vergleich): Katalysatorkombination N (160 cm), O (60 cm) P (70 cm) | 60 g/Nm$^3$ | 110,3 Gew.-% | 444 °C 65 cm (1. Lage) |
| 15 (Erfindungsgemäß): Katalysatorkombination Q (160cm)R(60cm) S (70 cm) | 60 g/Nm$^3$ | 113,5 Gew.-% | 442 °C 65 cm (1. Lage) |

Wie aus Tabelle 3 ersichtlich, zeigt der erfindungsgemäße 3-Lagen-Katalysator gemäß Beispiel 14, eine erheblich bessere PSA-Ausbeute.

**Patentansprüche**

1. Mehrlagen-Katalysator zur Herstellung von Phthalsäureanhydrid durch Gasphasenoxidation von o-Xylol und/oder Naphtalin, enthaltend eine erste, zur Gaseintrittsseite hin gelegene Katalysatorlage, eine zweite, näher zur Gasaustrittsseite hin gelegene Katalysatorlage und eine dritte, noch näher zur oder an der Gasaustrittsseite hin gelegene Katalysatorlage, wobei die Katalysatorlagen unterschiedlich zusammengesetzt sind und jeweils eine Aktivmasse, die TiO$_2$ mit einem Gehalt an Na von weniger als 0,3 Gew.-% enthält, aufweisen, und wobei der Aktivmassegehalt von der ersten Katalysatorlage zur dritten Katalysatorlageabnimmt, mit der Maßgabe, dass

    a) die erste Katalysatorlage einen Aktivmassegehalt zwischen etwa 7 und 12 Gew.-% aufweist,
    b) die zweite Katalysatorlage einen Aktivmassegehalt im Bereich zwischen 6 und 11 Gew.-% aufweist, wobei der Aktivmassegehalt der zweiten Katalysatorlage kleiner oder gleich dem Aktivmassegehalt der ersten Katalysatorlage ist, und
    c) die dritte Katalysatorlage einen Aktivmassegehalt im Bereich zwischen 5 und 10 Gew.-% aufweist, wobei der Aktivmassegehalt der dritten Katalysatorlage kleiner oder gleich dem Aktivmassegehalt der zweiten Katalysatorlage ist.

2. Mehrlagen-Katalysator nach Anspruch 1, **dadurch gekennzeichnet, dass** die erste Katalysatorlage einen Aktivmassegehalt zwischen etwa 8 und 11 Gew.-% aufweist.

3. Mehrlagen-Katalysator nach einem der vorstehenden Ansprüche, **dadurch gekennzeichnet, dass** die zweite Katalysatorlage einen Aktivmassegehalt zwischen etwa 7 und 10 Gew.-% aufweist.

4. Mehrlagen-Katalysator nach einem der vorstehenden Ansprüche, **dadurch gekennzeichnet, dass** die dritte Katalysatorlage einen Aktivmassegehalt zwischen etwa 6 und 9 Gew.-% aufweist.

5. Mehrlagen-Katalysator nach einem der vorstehenden Ansprüche, **dadurch gekennzeichnet, dass** der Aktivmassegehalt von Katalysatorlage zu Katalysatorlage in Gasstromrichtung abnimmt.

6. Mehrlagen-Katalysator nach einem der vorstehenden Ansprüche, **dadurch gekennzeichnet, dass** der Cs-Gehalt (berechnet als Cs) von Katalysatorlage zu Katalysatorlage in Gasstromrichtung abnimmt.

7. Mehrlagen-Katalysator nach einem der vorstehenden Ansprüche, **dadurch gekennzeichnet, dass** nur die letzte Katalysatorlage Phosphor, insbesondere zwischen 0,01 und 0,5 Gew.-%, weiter bevorzugt zwischen 0,1 und 0,4 Gew.-% Phosphor (berechnet als Phosphor) enthält.

8. Mehrlagen-Katalysator nach einem der vorstehenden Ansprüche, **dadurch gekennzeichnet, dass** die Katalysatoraktivität der Katalysatorlage zur Gaseintrittsseite hin geringer ist als die Katalysatoraktivität der Katalysatorlage zur Gasaustrittsseite hin.

9. Mehrlagen-Katalysator nach einem der vorstehenden Ansprüche, **dadurch gekennzeichnet, dass** die BET-Oberfläche der ersten Katalysatorlage geringer ist als die BET-Oberfläche der dritten Katalysatorlage.

**10.** Mehrlagen-Katalysator nach einem der vorstehenden Ansprüche, **dadurch gekennzeichnet, dass** die BET-Oberfläche der ersten und der zweiten Katalysatorlage gleich sind, während die BET-Oberfläche der dritten Katalysatorlage demgegenüber größer ist.

**11.** Mehrlagen-Katalysator nach einem der vorstehenden Ansprüche, **dadurch gekennzeichnet, dass** die BET-Oberfläche der ersten und zweiten Katalysatorlage jeweils zwischen etwa 15 und 25 $m^2$/g liegt, und die BET-Oberfläche der dritten Katalysatorlage zwischen etwa 25 und 45 $m^2$/g liegt.

**12.** Mehrlagen-Katalysator nach einem der vorstehenden Ansprüche, **dadurch gekennzeichnet, dass** die erste, zur Gaseintrittsseite hin gelegene Katalysatorlage einen Längenanteil, bezogen auf die Gesamtlänge des Katalysatorbettes, von mindestens 40%, insbesondere mindestens 45%, besonders bevorzugt mindestens 50%, aufweist.

**13.** Mehrlagen-Katalysator nach einem der vorstehenden Ansprüche, **dadurch gekennzeichnet, dass** der Anteil der ersten Katalysatorlage an der Gesamtlänge des Katalysatorbettes zwischen 40 und 70%, insbesondere zwischen 40 und 55%, besonders bevorzugt zwischen 40 und 52%, liegt.

**14.** Mehrlagen-Katalysator nach einem der vorstehenden Ansprüche, **dadurch gekennzeichnet, dass** der Anteil der zweiten Katalysatorlage an der Gesamtlänge des Katalysatorbettes zwischen etwa 10 und 40%, insbesondere zwischen etwa 10 und 30%, liegt.

**15.** Mehrlagen-Katalysator nach einem der vorstehenden Ansprüche, **dadurch gekennzeichnet, dass** das Verhältnis der Länge der dritten Katalysatorlage zur Länge der zweiten Katalysatorlage zwischen etwa 1 und 2, insbesondere zwischen 1,2 und 1,7, besonders bevorzugt zwischen 1,3 und 1,6, liegt.

**16.** Mehrlagen-Katalysator nach einem der vorstehenden Ansprüche, **dadurch gekennzeichnet, dass** mindestens etwa 40%, insbesondere mindestens etwa 50%, besonders bevorzugt mindestens 60% des gesamten Porenvolumens des eingesetzten $TiO_2$ durch Poren mit einem Radius zwischen 60 und 400 nm gebildet werden.

**17.** Mehrlagen-Katalysator nach einem der vorstehenden Ansprüche, **dadurch gekennzeichnet, dass** bis zu 75%, insbesondere bis zu 70% des gesamten Porenvolumens des eingesetzten $TiO_2$ durch Poren mit einem Radius zwischen 60 und 400 nm gebildet werden.

**18.** Mehrlagen-Katalysator nach einem der vorstehenden Ansprüche, **dadurch gekennzeichnet, dass** die katalytisch aktive Masse im Fließ- bzw. Wirbelbett aufgebracht wird.

**19.** Mehrlagen-Katalysator nach einem der vorstehenden Ansprüche, **dadurch gekennzeichnet, dass** weniger als etwa 30%, insbesondere weniger als 22% des gesamten Porenvolumens des eingesetzten $TiO_2$ durch Poren mit einem Radius von mehr als 400 nm gebildet werden.

**20.** Mehrlagen-Katalysator nach einem der vorstehenden Ansprüche, **dadurch gekennzeichnet, dass** etwa 17 bis 27% des gesamten Porenvolumens des eingesetzten $TiO_2$ durch Poren mit einem Radius von mehr als 400 nm gebildet werden.

**21.** Mehrlagen-Katalysator nach einem der vorstehenden Ansprüche, **dadurch gekennzeichnet, dass** etwa 50 bis 75%, insbesondere etwa 50 bis 70%, besonders bevorzugt 50 bis 65% des gesamten Porenvolumens des eingesetzten $TiO_2$ durch Poren mit einem Radius von 60 bis 400 nm gebildet werden.

**22.** Mehrlagen-Katalysator nach einem der vorstehenden Ansprüche, **dadurch gekennzeichnet, dass** weniger als 30%, insbesondere weniger als 20% des gesamten Porenvolumens des eingesetzten $TiO_2$ durch Poren mit einem Radius von 3,7 bis 60 nm gebildet werden.

**23.** Mehrlagen-Katalysator nach einem der vorstehenden Ansprüche, **dadurch gekennzeichnet, dass** etwa 10 bis 30% des gesamten Porenvolumens des eingesetzten $TiO_2$ durch Poren mit einem Radius von 3,7 bis 60 nm gebildet werden.

**24.** Mehrlagen-Katalysator nach einem der vorstehenden Ansprüche, **dadurch gekennzeichnet, dass** der $D_{90}$-Wert des eingesetzten $TiO_2$ zwischen etwa 0,5 und 20 $\mu$m, insbesondere zwischen etwa 1 und 10 $\mu$m, besonders bevorzugt zwischen etwa 2 und 5 $\mu$m liegt.

**25.** Mehrlagen-Katalysator nach einem der vorstehenden Ansprüche, **dadurch gekennzeichnet, dass** weniger als 10%, insbesondere weniger als 5% des gesamten Porenvolumens des eingesetzten $TiO_2$ durch Mikroporen mit einem Porenradius von weniger als 3,7 nm vorhanden sind.

**26.** Mehrlagen-Katalysator nach einem der vorstehenden Ansprüche, **dadurch gekennzeichnet, dass** 8 Gew.-% oder mehr der katalytisch aktiven Masse, insbesondere zwischen etwa 8 und 15 Gew.-% an Vanadium, berechnet als Vanadiumpentoxid, vorliegen.

**27.** Mehrlagen-Katalysator nach einem der vorstehenden Ansprüche, **dadurch gekennzeichnet, dass** mindestens 0,05 Gew.-% der katalytisch aktiven Masse aus mindestens einem Alkalimetall, berechnet als Alkalimetall(e), gebildet wird.

**28.** Mehrlagen-Katalysator nach einem der vorstehenden Ansprüche, **dadurch gekennzeichnet, dass** als Kleber für die katalytisch aktive Masse ein organisches Polymer oder Copolymer, insbesondere ein Vinylacetatcopolymer, verwendet wird.

**29.** Mehrlagen-Katalysator nach einem der vorstehenden Ansprüche, **dadurch gekennzeichnet, dass** der Katalysator mindestens 24 Stunden bei > 390°C, bevorzugt zwischen 24 und 72 Stunden bei ≥ 400°C, in einem $O_2$-haltigen Gas, insbesondere in Luft, calciniert bzw. konditioniert wird.

**30.** Mehrlagen-Katalysator nach einem der vorstehenden Ansprüche, **dadurch gekennzeichnet, dass** Niob in einer Menge von 0,1 bis 2 Gew.-%, insbesondere 0,5 bis 1 Gew.-% der katalytisch aktiven Masse vorhanden ist.

**31.** Mehrlagen-Katalysator nach einem der vorstehenden Ansprüche, **dadurch gekennzeichnet, dass** nur eine $TiO_2$-Quelle verwendet wird.

**32.** Mehrlagen-Katalysator nach einem der Ansprüche 1-6 oder 8-31, **dadurch gekennzeichnet, dass** kein Phosphor in der aktiven Masse enthalten ist.

**33.** Verfahren zur Herstellung von Phthalsäureanhydrid durch Gasphasenoxidation von o-Xylol und/oder Naphthalin, wobei ein drei- oder mehrschichtiger Katalysator nach einem oder mehreren der vorstehenden Ansprüche verwendet wird.

**34.** Verwendung eines Mehrlagen-Katalysators nach einem der vorstehenden Ansprüche zur Herstellung von Phthalsäureanhydrid durch Gasphasenoxidation von o-Xylol und/oder Naphthalin.

**Claims**

**1.** Multi-layer catalyst for preparing phthalic anhydride by gas phase oxidation of o-xylene and/or naphthalene, comprising a first catalyst zone disposed toward a gas inlet side, a second catalyst zone disposed closer to the gas outlet side and a third catalyst zone disposed even closer to or at the gas outlet side, the catalyst zones having different compositions and in each case an active composition comprising $TiO_2$ with a content of Na of less than 0.3% by weight, and the active composition content decreasing from the first catalyst zone to the third catalyst zone, with the proviso that

a) the first catalyst zone has an active composition content between about 7 and 12% by weight,
b) the second catalyst zone has an active composition content in the range between 6 and 11% by weight, the active composition content of the second catalyst zone being less than or equal to the active composition content of the first catalyst zone, and
c) the third catalyst zone has an active composition content in the range between 5 and 10% by weight, the active composition content of the third catalyst zone being less than or equal to the active composition content of the second catalyst zone.

**2.** Multi-layer catalyst according to claim 1, **characterized in that** the first catalyst zone has an active composition content between about 8 and 11% by weight.

**3.** Multi-layer catalyst according to one of the preceding claims, **characterized in that** the second catalyst zone has an active composition content between about 7 and 10% by weight.

4. Multi-layer catalyst according to one of the preceding claims, **characterized in that** the third catalyst zone has an active composition content between about 6 and 9% by weight.

5. Multi-layer catalyst according to one of the preceding claims, **characterized in that** the active composition content decreases from catalyst zone to catalyst zone in the direction of the gas flow.

6. Multi-layer catalyst according to one of the preceding claims, **characterized in that** the Cs-content (calculated as Cs) decreases from catalyst zone to catalyst zone in the direction of the gas flow.

7. Multi-layer catalyst according to one of the preceding claims, **characterized in that** only the last catalyst layer contains phosphorous, in particular between 0.01 and 0.5% by weight, further preferred between 0.1 and 0.4% by weight phosphorous (calculated as phosphorous).

8. Multi-layer catalyst according to one of the preceding claims, **characterized in that** the catalyst activity of the catalyst zone toward the gas inlet side is lower than the catalyst activity of the catalyst zone toward the gas outlet side.

9. Multi-layer catalyst according to one of the preceding claims, **characterized in that** the BET surface area of the first catalyst zone is lower than the BET surface area of the third catalyst zone.

10. Multi-layer catalyst according to one of the preceding claims, **characterized in that** the BET surface area of the first and of the second catalyst zone is the same, while the BET surface area of the third catalyst zone is greater in comparison.

11. Multi-layer catalyst according to one of the preceding claims, **characterized in that** the BET surface area of the first and second catalyst zone is in each case between about 15 and 25 $m^2/g$, and the BET surface area of the third catalyst zone is between about 25 and 45 $m^2/g$.

12. Multi-layer catalyst according to one of the preceding claims, **characterized in that** the first catalyst zone disposed toward the gas inlet side has a length fraction, based on the total length of the catalyst bed, of at least 40%, in particular at least 45%, more preferably at least 50%.

13. Multi-layer catalyst according to one of the preceding claims, **characterized in that** the proportion of the first catalyst zone in the total length of the catalyst bed is between 40 and 70%, in particular between 40 and 55%, more preferably between 40 and 52%.

14. Multi-layer catalyst according to one of the preceding claims, **characterized in that** the proportion of the second catalyst zone in the total length of the catalyst bed is between about 10 and 40%, in particular between about 10 and 30%.

15. Multi-layer catalyst according to one of the preceding claims, **characterized in that** the ratio of the length of the third catalyst zone to the length of the second catalyst zone is between about 1 and 2, in particular between 1.2 and 1.7, more preferably between 1.3 and 1.6.

16. Multi-layer catalyst according to one of the preceding claims, **characterized in that** at least about 40%, in particular at least about 50%, more preferably at least 60%, of the total pore volume of the $TiO_2$ used is formed by pores having a radius between 60 and 400 nm.

17. Multi-layer catalyst according to one of the preceding claims, **characterized in that** up to 75%, in particular up to 70% of the total pore volume of the $TiO_2$ used is formed by pores having a radius between 60 and 400 nm.

18. Multi-layer catalyst according to one of the preceding claims, **characterized in that** the catalytically active composition is applied in a moving bed or fluidized bed.

19. Multi-layer catalyst according to one of the preceding claims, **characterized in that** less than about 30%, in particular less than 22%, of the total pore volume of the $TiO_2$ used is formed by pores having a radius of more than 400 nm.

**20.** Multi-layer catalyst according to one of the preceding claims, **characterized in that** about 17 to 27% of the total pore volume of the $TiO_2$ used is formed by pores having a radius of more than 400 nm.

**21.** Multi-layer catalyst according to one of the preceding claims, **characterized in that** about 50 to 75%, in particular about 50 to 70%, more preferably 50 to 65% of the total pore volume of the $TiO_2$ is formed by pores having a radius of 60 to 400 nm.

**22.** Multi-layer catalyst according to one of the preceding claims, **characterized in that** less than 30%, in particular less than 20%, of the total pore volume of the $TiO_2$ used is formed by pores having a radius of 3.7 to 60 nm.

**23.** Multi-layer catalyst according to one of the preceding claims, **characterized in that** about 10 to 30% of the total pore volume of the $TiO_2$ used is formed by pores having a radius of 3.7 to 60 nm.

**24.** Multi-layer catalyst according to one of the preceding claims, **characterized in that** the $D_{90}$ value of the $TiO_2$ used is between about 0.5 and 20 $\mu$m, in particular between about 1 and 10 $\mu$m, more preferably between about 2 and 5 $\mu$m.

**25.** Multi-layer catalyst according to one of the preceding claims, **characterized in that** less than 10%, in particular less than 5%, of the total pore volume of the $TiO_2$ used is present by virtue of micropores having a pore radius of less than 3.7 nm.

**26.** Multi-layer catalyst according to one of the preceding claims, **characterized in that** 8% by weight or more of the catalytically active composition, in particular between about 8 and 15% by weight of vanadium, calculated as vanadium pentoxide, is present.

**27.** Multi-layer catalyst according to one of the preceding claims, **characterized in that** at least 0.05% by weight of the catalytically active composition is formed from at least one alkali metal, calculated as alkali metal(s).

**28.** Multi-layer catalyst according to one of the preceding claims, **characterized in that** the adhesive used for the catalytically active composition is an organic polymer or copolymer, in particular a vinyl acetate copolymer.

**29.** Multi-layer catalyst according to one of the preceding claims, **characterized in that** the catalyst is calcined or conditioned in an $O_2$-containing gas, in particular in air, at > 390°C for at least 24 hours, preferably at $\geq$ 400°C for between 24 and 72 hours.

**30.** Multi-layer catalyst according to one of the preceding claims, **characterized in that** niobium is present in an amount of 0.1 to 2% by weight, in particular 0.5 to 1% by weight, of the catalytically active composition.

**31.** Multi-layer catalyst according to one of the preceding claims, **characterized in that** only one $Ti_2$ source is used.

**32.** Multi-layer catalyst according to one of the preceding claims, **characterized in that** no phosphorus is present in the active composition.

**33.** Process for preparing phthalic anhydride by gas phase oxidation of o-xylene and/or naphthalene, by using a three-layer or multi-layer catalyst according to one or more of the preceding claims.

**34.** Use of a catalyst according to one of the preceding claims for preparing phtalic anhydride by gas phase oxidation of o-xylene and/or napthalene.

**Revendications**

**1.** Catalyseur multicouche pour la fabrication d'anhydride d'acide phtalique par oxydation en phase gazeuse d'o-xylène et/ou de naphtalène, contenant une première couche de catalyseur située vers le côté d'entrée du gaz, une deuxième couche de catalyseur située plus près du côté de sortie du gaz et une troisième couche de catalyseur située encore plus près de ou sur le côté de sortie du gaz, dans lequel les couches de catalyseur sont de composition différente et présentent chacune une masse active qui contient du $TiO_2$ avec une teneur en Na inférieure à 0,3 % en poids, et dans lequel la teneur en masse active diminue de la première couche de catalyseur à la troisième couche de catalyseur, à condition que

a) la première couche de catalyseur présente une teneur en masse active comprise entre environ 7 et 12 % en poids,
b) la deuxième couche de catalyseur présente une teneur en masse active dans la plage comprise entre 6 et 11% en poids, dans lequel la teneur en masse active de la deuxième couche de catalyseur est inférieure ou égale à la teneur en masse active de la première couche de catalyseur, et
c) la troisième couche de catalyseur présente une teneur en masse active dans la plage comprise entre 5 et 10 % en poids, dans lequel la teneur en masse active de la troisième couche de catalyseur est inférieure ou égale à la teneur en masse active de la deuxième couche de catalyseur.

2. Catalyseur multicouche selon la revendication 1, **caractérisé en ce que** la première couche de catalyseur présente une teneur en masse active comprise entre environ 8 et 11 % en poids.

3. Catalyseur multicouche selon l'une quelconque des revendications précédentes, **caractérisé en ce que** la deuxième couche de catalyseur présente une teneur en masse active comprise entre environ 7 et 10 % en poids.

4. Catalyseur multicouche selon l'une quelconque des revendications précédentes, **caractérisé en ce que** la troisième couche de catalyseur présente une teneur en masse active comprise entre environ 6 et 9 % en poids.

5. Catalyseur multicouche selon l'une quelconque des revendications précédentes, **caractérisé en ce que** la teneur en masse active diminue d'une couche de catalyseur à une autre dans le sens d'écoulement du gaz.

6. Catalyseur multicouche selon l'une quelconque des revendications précédentes, **caractérisé en ce que** la teneur en Cs (calculée en tant que Cs) d'une couche de catalyseur à une autre diminue dans le sens d'écoulement du gaz.

7. Catalyseur multicouche selon l'une quelconque des revendications précédentes, **caractérisé en ce que** seule la dernière couche de catalyseur contient du phosphore, notamment entre 0,01 à 0,5 % en poids, de manière davantage préférée entre 0,1 et 0,4 % en poids de phosphore (calculé en tant que phosphore).

8. Catalyseur multicouche selon l'une quelconque des revendications précédentes, **caractérisé en ce que** l'activité de catalyseur de la couche de catalyseur est plus faible vers le côté d'entrée du gaz que l'activité de catalyseur de la couche de catalyseur vers le côté de sortie du gaz.

9. Catalyseur multicouche selon l'une quelconque des revendications précédentes, **caractérisé en ce que** la surfaces BET de la première couche de catalyseur est plus faible que la surface BET de la troisième couche de catalyseur.

10. Catalyseur multicouche selon l'une quelconque des revendications précédentes, **caractérisé en ce que** la surface BET de la première et de la deuxième couche de catalyseur est égale tandis que la surface BET de la troisième couche de catalyseur est supérieure par rapport à celle-ci.

11. Catalyseur multicouche selon l'une quelconque des revendications précédentes, **caractérisé en ce que** la surface BET de la première et deuxième couche de catalyseur est à chaque fois comprise entre environ 15 et 25 m$^2$/g, et la surface BET de la troisième couche de catalyseur est comprise entre environ 25 et 45 m$^2$/g.

12. Catalyseur multicouche selon l'une quelconque des revendications précédentes, **caractérisé en ce que** la première couche de catalyseur située vers le côté d'entrée du gaz présente une proportion de longueur, par rapport à la longueur totale du lit de catalyseur,
d'au moins 40 %, notamment d'au moins 45 %, de manière particulièrement préférée d'au moins 50 %.

13. Catalyseur multicouche selon l'une quelconque des revendications précédentes, **caractérisé en ce que** la proportion de la première couche de catalyseur sur la longueur totale du lit de catalyseur se situe entre 40 et 70 %, notamment entre 40 et 55 %, de manière particulièrement préférée entre 40 et 52 %.

14. Catalyseur multicouche selon l'une quelconque des revendications précédentes, **caractérisé en ce que** la proportion de la deuxième couche de catalyseur sur la longueur totale du lit de catalyseur se situe entre environ 10 et 40 %, notamment entre environ 10 et 30 %.

15. Catalyseur multicouche selon l'une quelconque des revendications précédentes, **caractérisé en ce que** le rapport entre la longueur de la troisième couche de catalyseur et la longueur de la deuxième couche de catalyseur se situe entre environ 1 et 2, notamment entre 1,2 et 1,7, de manière particulièrement préférée entre 1,3 et 1,6.

**16.** Catalyseur multicouche selon l'une quelconque des revendications précédentes, **caractérisé en ce qu'**au moins environ 40 %, notamment au moins environ 50 %, de manière particulièrement préférée au moins 60 % du volume poreux total du $TiO_2$ utilisé sont formés par des pores avec un rayon compris entre 60 et 400 nm.

**17.** Catalyseur multicouche selon l'une quelconque des revendications précédentes, **caractérisé en ce que** jusqu'à 75 %, notamment jusqu'à 70% du volume poreux total du $TiO_2$ utilisé sont formés par des pores avec un rayon compris entre 60 et 400 nm.

**18.** Catalyseur multicouche selon l'une quelconque des revendications précédentes, **caractérisé en ce que** la masse active du point de vue catalytique est appliquée dans un lit mobile ou fluidisé.

**19.** Catalyseur multicouche selon l'une quelconque des revendications précédentes, **caractérisé en ce que** moins d'environ 30 %, notamment moins de 22 % du volume poreux total du $TiO_2$ utilisé sont formés par des pores avec un rayon supérieur à 400 nm.

**20.** Catalyseur multicouche selon l'une quelconque des revendications précédentes, **caractérisé en ce qu'**environ 17 à 27 % du volume poreux total du $TiO_2$ utilisé sont formés par des pores avec un rayon supérieur à 400 nm.

**21.** Catalyseur multicouche selon l'une quelconque des revendications précédentes, **caractérisé en ce qu'**environ 50 à 75 %, notamment environ 50 à 70 %, de manière particulièrement préférée 50 à 65 % du volume poreux total du $TiO_2$ utilisé sont formés par des pores avec un rayon de 60 à 400 nm.

**22.** Catalyseur multicouche selon l'une quelconque des revendications précédentes, **caractérisé en ce que** moins de 30 %, notamment moins de 20 % du volume poreux total du $TiO_2$ utilisé sont formés par des pores avec un rayon de 3,7 à 60 nm.

**23.** Catalyseur multicouche selon l'une quelconque des revendications précédentes, **caractérisé en ce qu'**environ 10 à 30 % du volume poreux total du $TiO_2$ utilisé sont formés par des pores avec un rayon de 3,7 à 60 nm.

**24.** Catalyseur multicouche selon l'une quelconque des revendications précédentes, **caractérisé en ce que** la valeur $D_{90}$ du $TiO_2$ utilisé se situe entre environ 0,5 et 20 $\mu$m, notamment entre environ 1 et 10 $\mu$m, de manière particu-lièrement préférée entre environ 2 et 5 $\mu$m.

**25.** Catalyseur multicouche selon l'une quelconque des revendications précédentes, **caractérisé en ce que** moins de 10 %, notamment moins de 5 % du volume poreux total du $TiO_2$ utilisé sont présents par des micropores avec un rayon de pore inférieur à 3,7 nm.

**26.** Catalyseur multicouche selon l'une quelconque des revendications précédentes, **caractérisé en ce que** 8% en poids ou plus de la masse active du point de vue catalytique, notamment entre environ 8 et 15 % en poids de vanadium, calculé en tant que pentoxyde de vanadium, sont présents.

**27.** Catalyseur multicouche selon l'une quelconque des revendications précédentes, **caractérisé en ce qu'**au moins 0,05 % en poids de la masse active du point de vue catalytique est formé d'au moins un métal alcalin, calculé en tant que métal(métaux) alcalin(s).

**28.** Catalyseur multicouche selon l'une quelconque des revendications précédentes, **caractérisé en ce qu'**un polymère ou copolymère organique, notamment un copolymère d'acétate de vinyle, est utilisé en tant qu'adhésif pour la masse active du point de vue catalytique.

**29.** Catalyseur multicouche selon l'une quelconque des revendications précédentes, **caractérisé en ce que** le catalyseur est calciné ou conditionné au moins pendant 24 heures à > 390°C, de préférence entre 24 et 72 heures à $\geq$ 400°C, dans un gaz contenant de l'$O_2$, notamment dans l'air.

**30.** Catalyseur multicouche selon l'une quelconque des revendications précédentes, **caractérisé en ce que** du niobium est présent en une quantité de 0,1 à 2 % en poids, notamment 0,5 à 1% en poids de la masse active du point de vue catalytique.

**31.** Catalyseur multicouche selon l'une quelconque des revendications précédentes, **caractérisé en ce que** seule une source de TiO$_2$ est utilisée.

**32.** Catalyseur multicouche selon l'une quelconque des revendications 1 à 6 ou 8 à 31, **caractérisé en ce qu'**aucun phosphore n'est contenu dans la masse active.

**33.** Procédé de fabrication d'anhydride d'acide phtalique par oxydation en phase gazeuse d'o-xylène et/ou de naphtalène, dans lequel un catalyseur à trois couches ou multicouche selon l'une quelconque ou plusieurs des revendications précédentes est utilisé.

**34.** Utilisation d'un catalyseur multicouche selon l'une quelconque des revendications précédentes pour la fabrication d'anhydride d'acide phtalique par oxydation en phase gazeuse d'o-xylène et/ou de naphtalène.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1082317 B1
- EP 1084115 B1
- EP 1084115 A
- DE 10040827 A1
- EP 0964744 B1
- DE 2159441 A
- DE 1642938 A
- DE 1769998 A
- DE 2106796
- US 2035606 A
- DE 1280756
- DE 19828583
- DE 19709589
- EP 744214 A
- WO 9800778 A
- EP 714700 A
- WO 9837967 A
- WO 9961433 A

**In der Beschreibung aufgeführte Nicht-Patentliteratur**

- Phtalic Acid and Derivatives. **K. Towae ; W. Enke ; R. Jäckh ; N. Bhargana.** Ullmann's Encyclopedia of Industrial Chemistry. 1992, vol. A. 20, 181
- *J. Am. Chem. Soc.,* 1938, vol. 60, 309